# EUROPEAN PATENT APPLICATION

(11) **EP 3 006 428 A1**
(43) Date of publication of application: **13.04.2016**
(21) Application number: 14808319.9
(22) Date of filing: 03.06.2014
(51) Int. Cl.: C07D 213/36, A61K 31/4402, A61P 35/00

(54) **NOVEL MALEIC ACID DERIVATIVE, PRODUCTION METHOD FOR SAME AND ANTI-CANCER COMPOSITION COMPRISING SAME**

(30) Priority: 05.06.2013 KR 20130065015
(71) Applicant: Medicinal Bioconvergence Research Center, Suwon-si, Gyeonggi-do 443-270 (KR)
(72) Inventor: KIM, Sunghoon, Seoul 135-859 (KR); KWON, Nam Hoon, Seoul 151-780 (KR); KIM, Dae Gyu, Seoul 135 859 (KR); LEE, Sunkyung, Daejeon 305 707 (KR); SONG, Jong Hwan, Okcheon-gun Chungcheongbuk-do 373-863 (KR)
(74) Representative: Isarpatent
(86) International application number: PCT/KR2014/004933
(87) International publication number: WO 2014/196784

(57) **Abstract**

The present invention relates to a novel anticancer composition, and more specifically relates to a maleic acid derivative compound of Chemical Formula 1, to a production method for the compound, and to a pharmaceutical composition comprising the compound. The maleic acid derivative of the present invention suppresses the expression of AIMP2-DX2 and hence selectively suppresses the growth of cancer cell lines without acting on normal cells, and thus pharmaceutical compositions containing the maleic acid derivative of the present invention and pharmaceutically acceptable salts thereof as active ingredients can be used to prevent and treat cancer.

## Description

### [Technical Field]

The present application claims priority from Korean Patent Application No. 10-2013-0065015 filed on 5 June 2013, the disclosure of which is hereby incorporated herein by reference in its entirety.

The present invention relates to a novel maleic acid derivative or a pharmaceutically acceptable salt thereof, a method for preparing the same, and a pharmaceutical composition for preventing or treating cancer, comprising the same as an active ingredient.

### [Background Art]

Aminoacyl-tRNA synthetase (ARS) plays an important role in linking a specific amino acid to a tRNA molecule, leading to protein synthesis. In higher animals, nine classes of ARSs have been known to bind with three classes of ARS-interacting multi functional proteins (AIMPs) to form multi-synthetases complexes (MSCs). AIMP2, which is a multifunctional protein that maintains structures of MSCs and performs various functions in response to stress signals, acts as a tumor suppressor by leading to apoptosis under TGF-β, TNF-α, and DNA damage signals and maintaining the stability of p53 under DNA damage signals including UV and the like. AIMP2-DX2, which is an exon 2-deleted splicing variant of AIMP2, competitively inhibits the binding of AIMP2 and p53 to inhibit the pro-apoptotic activity of AIMP2 (Choi JW, et al., PLOS GENETICS, 7(3):e1001351, 2011). The studies found that AIMP2-DX2 may cause cancer by inhibiting cancer inhibition of AIMP2, and AIMP2-DX2 mRNA exhibits a high expression level of 80% in tissues of lung cancer patients and AIMP2-DX2 is also highly expressed at 60% in lung cancer. In addition, the expression level of AIMP2-DX2 over AIMP2 showed a close correlation with the progress of lung cancer.

The AIMP2-DX2 protein is a variant of AIMP2 in which exon 2 is deleted from the AIMP2 protein sequence, while sequences of the AIMP2 protein (312aa version: AAC50391.1 or GI:1215669; 320aa version: AAH13630.1, GI:15489023, BC013630.1) are disclosed in the literatures (312 aa version: Nicolaides,N.C., et. al., Genomics 29 (2), 329-334 (1995)/ / 320 aa version: Generation and initial analysis of more than 15,000 full-length human and mouse cDNA sequences, Proc. Natl. Acad.Sci. U.S.A. 99 (26), 16899-16903 (2002)).

Anticancer encompasses the effect of both the prevention and treatment of cancer. Generally, cancer refers to the malignancy of cells which are not normally differentiated and have grown out of control since there is abnormality in the cycle stage of cells constituting human tissues. The types of cancer as used herein may include breast cancer, large intestine cancer, lung cancer, small cell lung cancer, gastric cancer, liver cancer, blood cancer, bone cancer, pancreatic cancer, skin cancer, head or neck cancer, cutaneous or intraocular melanoma, uterine cancer, ovarian cancer, rectal cancer, anal cancer, colon cancer, breast cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid carcinoma, adrenal cancer, soft tissue sarcoma, urethral cancer, penile cancer, prostate cancer, chronic or acute leukemia, lymphocyte lymphoma, bladder cancer, kidney or ureter cancer, renal cell carcinoma, renal pelvic carcinoma, CNS tumor, primary CNS lymphoma, spinal cord tumor, brain stem glioma, pituitary adenoma, and the like.

Regarding the prior art associated with the present invention, there is a patent literature (Korean Patent Application Publication No. 2006-0058014) that provides an exon 2-deleted variant of AIMP2, named AIMP2-DX2, which is specifically expressed in cancer tissues, and is used as a cancer diagnosis marker to diagnose cancer and inhibits AIMP2-DX2 to treat or prevent cancer. In addition, there are another patent literature (Korean Patent Application Publication No. 2009-0048382) regarding a composition for preventing and treating inflammatory disease, containing an AIMP2-DX2 inhibitor as an active ingredient; a composition for preventing and treating inflammatory disease, containing an expression vector inhibiting AIMP2-DX2 expression; and a method for screening a drug for preventing or treating inflammatory disease to screen a material inhibiting AIMP2-DX2 expression; and further another patent literature (Korean Patent Application Publication No. 2013-0016041) regarding an anticancer composition containing, as an active ingredient, an aniline derivative which is effective in the prevention and treatment of cancer since it effectively induces the inhibition of AIMP2-DX2 expression and the apoptosis of cancer cells.

### [Detailed Description of the Invention]

### [Technical Problem]

The present inventors, while searching for compounds capable of suppressing the growth of cancer cells by inhibiting expression of AIMP2-DX2, have found that maleic acid derivatives represented by Chemical Formula 1 herein inhibit the expression of AIMP2-DX2 and suppresses the proliferation of cancer cells, and thus are useful as an anticancer agent for cancer prevention and treatment.

Therefore, an aspect of the present invention is to provide a maleic acid derivative represented by Chemical Formula 1 below, or a pharmaceutically acceptable salt thereof: 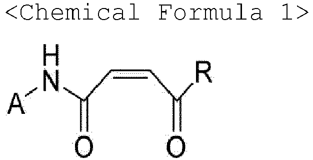 wherein,
A is 5- to 7-membered phenyl having a substituent group, or heteroaryl or heterocycloalkyl containing at least one heteroatom selected from the group consisting of N, 0, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group;
when R is OH, the phenyl having a substituent group is excluded from A, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
R is OH, OR¹, NH₂, NHR¹, NR¹R² 5- to 7-membered phenyl having a substituent group, or heteroaryl or heterocycloalkyl containing at least one heteroatom selected from the group consisting of N, O, and S and having a substituent group, wherein the substituent group is unsubstituted, halogen, OH, OR¹, nitro, nitrile, NH₂, NHR¹, NR¹R² COOH, COOR¹, CONH₂, CONHR¹, C₁₋C₅ straight- or branched-chain alkyl, phenyl, heteroaryl, or 5- to 7-membered heterocycloalkyl, where R¹ and R² each are C₁-C₅ straight- or branched-chain alkyl or phenyl.

Another aspect of the present invention is to provide a method for preparing a maleic acid derivative represented by Chemical Formula 1 above.

Still another aspect of the present invention is to provide a pharmaceutical composition for preventing and treating cancer based on the inhibition of AIMP2-DX2 expression, the composition comprising, as an active ingredient, a maleic acid derivative represented by Chemical Formula 1 above or a pharmaceutically acceptable salt thereof.

Still another aspect of the present invention is to provide a method for preventing or treating cancer, the method comprising administering the maleic acid derivative represented by Chemical Formula 1 or the pharmaceutically acceptable salt thereof to a subject in need thereof.

Still another aspect of the present invention is to provide a use of the maleic acid derivative represented by Chemical Formula 1 or the pharmaceutically acceptable salt thereof for the preparation of an agent for cancer prevention or treatment.

### [Technical Solution]

In accordance with an aspect of the present invention, there is provided a maleic acid derivative represented by Chemical Formula 1 below or a pharmaceutically acceptable salt thereof: 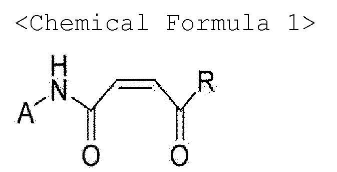 wherein,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group;
when R is OH, the phenyl having a substituent group is excluded from A, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
R is OH, OR¹, NH₂, NHR¹, NR¹R² 5- to 7-membered phenyl having a substituent group, or heteroaryl or heterocycloalkyl containing at least one heteroatom selected from the group consisting of N, O, and S and having a substituent group, wherein the substituent group is unsubstituted, halogen, OH, OR¹, nitro, nitrile, NH₂, NHR¹, NR¹R² COOH, COOR¹, CONH₂, CONHR¹, C₁₋C₅ straight- or branched-chain alkyl, phenyl, heteroaryl, or 5- to 7-membered heterocycloalkyl, where R¹ and R² each are C₁-C₅ straight- or branched-chain alkyl or phenyl.

In accordance with another aspect of the present invention, there is provided a method for preparing a maleic acid derivative represented by Chemical Formula 1 above.

In accordance with another aspect of the present invention, there is provided a pharmaceutical composition for preventing or treating cancer based on the inhibition of AIMP2-DX2 expression, the composition comprising a maleic acid derivative represented by Chemical Formula 1 below or a pharmaceutically acceptable salt thereof.

In accordance with still another aspect of the present invention, there is provided a method for preventing or treating cancer, the method comprising administering a maleic acid derivative represented by Chemical Formula 1 or a pharmaceutically acceptable salt thereof to a subject in need thereof.

In accordance with still another aspect of the present invention, there is provided a use of a maleic acid derivative represented by Chemical Formula 1 or a pharmaceutically acceptable salt thereof for the preparation of an agent for cancer prevention or treatment. 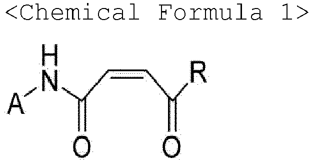 wherein,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group;
when R is OH, the phenyl having a substituent group is excluded from A, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
R is OH, OR¹, NH₂, NHR¹, NR¹R², 5- to 7-membered phenyl having a substituent group, or heteroaryl or heterocycloalkyl containing at least one heteroatom selected from the group consisting of N, O, and S and having a substituent group, wherein the substituent group is unsubstituted, halogen, OH, OR¹, nitro, nitrile, NH₂, NHR¹, NR¹R² COOH, COOR¹, CONH₂, CONHR¹, C₁₋C₅ straight- or branched-chain alkyl, phenyl, heteroaryl, or 5- to 7-membered heterocycloalkyl, where R¹ and R² each are C₁-C₅ straight- or branched-chain alkyl or phenyl.

Hereinafter, the present invention will be described in detail.

The present invention provides a maleic acid derivative represented by Chemical Formula 1 below or a pharmaceutically acceptable salt thereof: 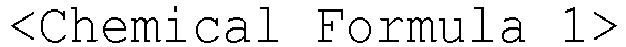 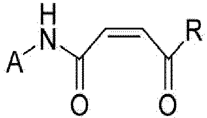 wherein,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group;
when R is OH, the phenyl having a substituent group is excluded from A, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
R is OH, OR¹, NH₂, NHR¹, NR¹R² 5- to 7-membered phenyl having a substituent group, or heteroaryl or heterocycloalkyl containing at least one heteroatom selected from the group consisting of N, O, and S and having a substituent group, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl or phenyl.

As used herein, the term "alkyl", unless otherwise indicated, refers to saturated, straight- or branched-chain hydrocarbon radicals.

As used herein, the term "cycloalkyl", unless otherwise indicated, refers to saturated hydrocarbon cycles.

More preferably, the compound of Chemical Formula 1 above is selected from the followings:
(*Z*)-4-oxo-4-(pyridin-2-ylamino)but-2-enoic acid;
(*Z*)-4-[(4-methylpyridin-2-yl)amino]-4-oxobut-2-enoic acid;
(*Z*)-4-oxo-4-(pyridin-3-ylamino)but-2-enoic acid;
(*Z*)-4-[(4,6-dimethylpyridin-2-yl)amino]-4-oxobut-2-enoic acid;
(*Z*)-4-[(5-chloropyridin-2-yl)amino]-4-oxobut-2-enoic acid;
(*Z*)-4-[(6-bromopyridin-3-yl)amino]-4-oxobut-2-enoic acid;
(*Z*)-4-[(5-methyl-1,3,4-thiadiazol-2-yl)amino]-4-oxobut-2-enoic acid;
(*Z*)-4-oxo-4-[(thiophen-2-ylmethyl)amino]but-2-enoic acid;
(*Z*)-4-[(3-cyanothiophen-2-yl)amino]-4-oxobut-2-enoic acid;
(*Z*)-4-(isoxazol-3-ylamino)-4-oxobut-2-enoic acid;
ethyl (*Z*)-4-[(3,4-dimethylphenyl)amino]-4-oxobut-2-enoate;
(*Z*)-4-oxo-4-(piperidin-1-yl)-N-(m-tolyl)but-2-enamide;
(*Z*)-*N*-(3,5-dimethylphenyl)-4-oxo-4-(piperidin-1-yl)but-2-enamide;
(*Z*)-*N*-(4-methylpyridin-2-yl)-4-oxo-4-(piperidin-1-yl)but-2-enamide;
(*Z*)-4-oxo-4-(piperidin-1-yl)-*N*-(pyridin-2-yl)but-2-enamide;
(*Z*)-4-oxo-4-(piperidin-1-yl)-*N*-(pyridin-3-yl)but-2-enamide;
(*Z*)-*N*-(4,6-dimethylpyridin-2-yl)-4-oxo-4-(piperidin-1-yl)but-2-enamide;
(*Z*)-4-morpholino-4-oxo-*N*-(*m*-tolyl)but-2-enamide;
(*Z*)-*N*-(3,5-dimethylphenyl)-4-morpholino-4-oxobut-2-enamide;
(*Z*)-*N*-(4-methylpyridin-2-yl)-4-morpholino-4-oxobut-2-enamide;
(*Z*)-4-oxo-4-(pyrrolidin-1-yl)-*N*-(*m*-tolyl)but-2-enamide;
(*Z*)-*N*-(3,5-dimethylphenyl)-4-oxo-4-(pyrrolidine-1-yl)but-2-enamide;
(*Z*)-*N*-(4-methylpyridin-2-yl)-4-oxo-4-(pyrrolidine-1-yl)but-2-enamide;
(*Z*)-*N*-(pyridin-2-yl)-4-oxo-4-(pyrrolidine-1-yl)but-2-enamide;
(*Z*)-*N*-(4-bromophenyl)-4-oxo-4-(piperidin-1-yl)but-2-enamide;
(*Z*)-*N*-(4-chlorophenyl)-4-oxo-4-(piperidin-1-yl)but-2-enamide;
(*Z*)-*N*-(4-bromophenyl)-4-morpholino-4-oxobut-2-enamide;
(*Z*)-*N*-(5-chloropyridin-2-yl)-4-(piperidin-1-yl)-4-oxobut-2-enamide;
(*Z*)-*N*-(6-bromopyridin-3-yl)-4-(piperidin-1-yl)-4-oxobut-2-enamide;
(Z)-*N*-(4-chlorophenyl)-4-oxo-4-(pyrrolidin-1-yl)but-2-enamide;
(*Z*)-*N*-(4-chlorophenyl)-4-morpholino-4-oxobut-2-enamide;
(*Z*)-*N*-(6-bromopyridin-3-yl)-4-morpholino-4-oxobut-2-enamide;
(*Z*)-*N*-(5-chloropyridin-2-yl)-4-morpholino-4-oxobut-2-enamide;
(*Z*)-*N*-(5-chloropyridin-2-yl)-4-oxo-4-(pyrrolidin-1-yl)but-2-enamide;
(*Z*)-*N*-(6-bromopyridin-3-yl)-4-oxo-4-(pyrrolidin-1-yl)but-2-enamide;
(*Z*)-*N*-(5-methyl-1,3,4-thiadiazol-2-yl)-4-oxo-4-(pyrrolidin-1-yl)but-2-enamide;
(*Z*)-4-oxo-4-(pyrrolidin-1-yl)-*N*-(thiophen-2-ylmethyl)but-2-enamide;
(*Z*)-4-oxo-4-(piperidin-1-yl)-*N*-(thiophen-2-ylmethyl)but-2-enamide;
(*Z*)-4-morpholino-4-oxo-N-(thiophen-2-ylmethyl)but-2-enamide;
(*Z*)-*N*-(3-cyanothiophen-2-yl)-4-oxo-4-(pyrrolidin-1-yl)but-2-enamide;
(*Z*)-*N*-(3-cyanothiophen-2-yl)-4-morpholino-4-oxobut-2-enamide;
(*Z*)-*N*-(3-cyanothiophen-2-yl)-4-oxo-4-(piperidin-1-yl)but-2-enamide;
(*Z*)-*N*-(3-cyanothiophen-2-yl)-4-oxo-4-(1*H*-pyrazol-1-yl)but-2-enamide;
(*Z*)-*N*-(isoxazol-3-yl)-4-oxo-4-(1*H*-pyrazol-1-yl)but-2-enamide;
(*Z*)-4-[(5-bromopyridin-2-yl)amino]-4-oxobut-2-enoic acid;
(*Z*)-*N*-(5-bromopyridin-2-yl)-4-morpholino-4-oxobut-2-enamide;
(*Z*)-*N*-(5-bromopyridin-2-yl)-4-oxo-4-(pyrrolidin-1-yl)but-2-enamide;
(*Z*)-*N*-(5-bromopyridin-2-yl)-4-oxo-4-(piperidin-1-yl)but-2-enamide;
(*Z*)-4-[(4-chloropyridin-2-yl)amino]-4-oxobut-2-enoic acid;
(*Z*)-*N*-(4-chloropyridin-2-yl)-4-morpholino-4-oxobut-2-enamide;
(*Z*)-*N*-(4-chloropyridin-2-yl)-4-oxo-4-(pyrrolidin-1-yl)but-2-enamide.
(*Z*)-*N*-(4-chloropyridin-2-yl)-4-oxo-4-(piperidin-1-yl)but-2-enamide;
(*Z*)-4-[(4-bromopyridin-2-yl)amino]-4-oxobut-2-enoic acid;
(*Z*)-*N*-(4-bromopyridin-2-yl)-4-morpholino-4-oxobut-2-enamide;
(*Z*)-*N*-(4-bromopyridin-2-yl)-4-oxo-4-(pyrrolidin-1-yl)but-2-enamide;
(*Z*)-4-morpholino-4-oxo-*N*-(4-phenylpyridin-2-yl)-but-2-enamide; and
(*Z*)-4-methyl-2-(4-morpholino-4-oxo-but-2-enamido)pyridine 1-oxide

In the pharmaceutical composition according to the present invention, the structural formulas of the compounds are summarized in [Table 1] below.

**[Table 1]**

| Example | Structure | Example | Structure |
|---|---|---|---|
| 1-1 | 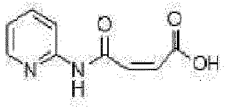 | 1-2 | 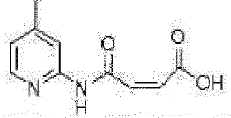 |
| 1-3 | 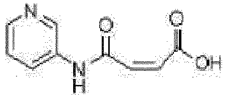 | 1-4 | 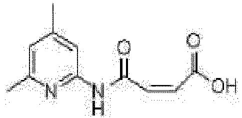 |
| 1-5 | 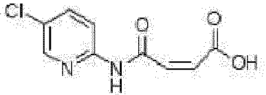 | 1-6 | 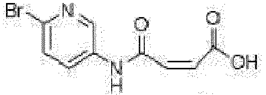 |
| 1-7 | 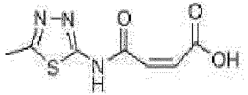 | 1-8 | 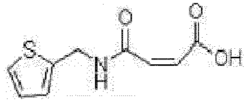 |
| 1-9 | 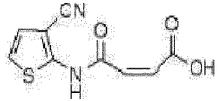 | 1-10 | 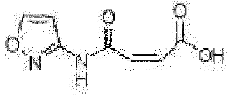 |
| 1-11 | 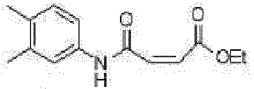 | 1-12 | 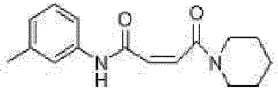 |
| 1-13 | 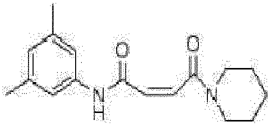 | 1-14 | 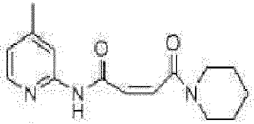 |
| 1-15 | 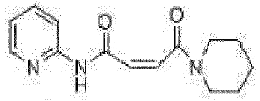 | 1-16 | 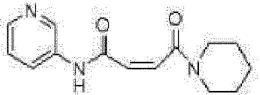 |
| 1-17 | 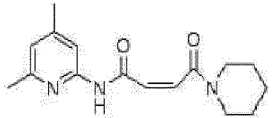 | 1-18 | 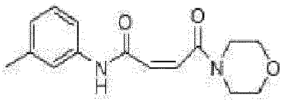 |
| 1-19 | 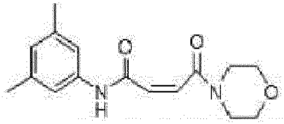 | 1-20 | 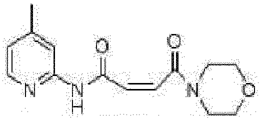 |
| 1-21 | 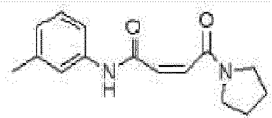 | 1-22 | 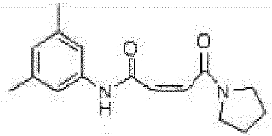 |
| 1-23 | 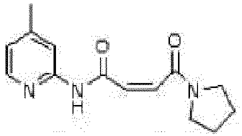 | 1-24 | 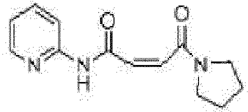 |
| 1-25 | 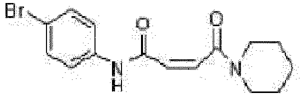 | 1-26 | 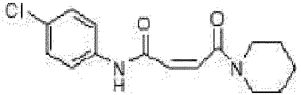 |
| 1-27 | 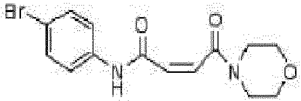 | 1-38 | 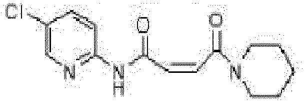 |
| 1-29 | 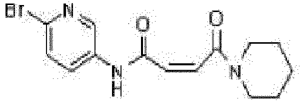 | 1-30 | 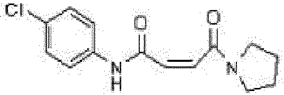 |
| 1-31 | 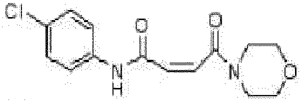 | 1-32 | 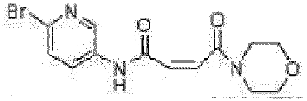 |
| 1-33 | 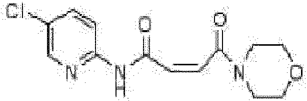 | 1-34 | 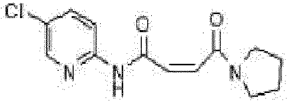 |
| 1-35 | 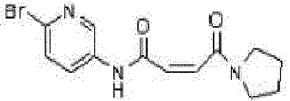 | 1-36 | 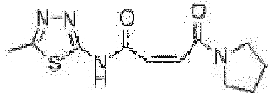 |
| 1-37 | 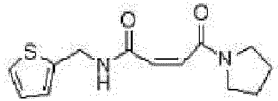 | 1-38 | 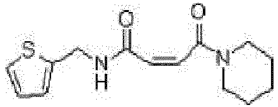 |
| 1-39 | 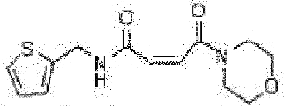 | 1-40 | 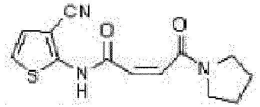 |
| 1-41 | 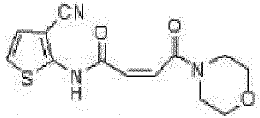 | 1-42 | 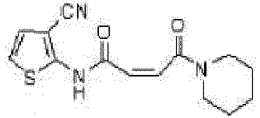 |
| 1-43 | 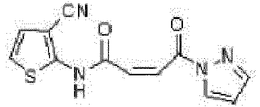 | 1-44 | 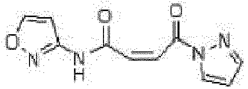 |
| 1-45 | 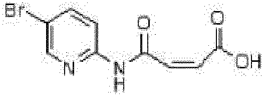 | 1-44 | 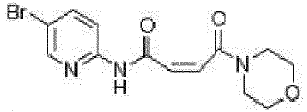 |
| 1-47 | 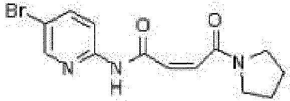 | 1-48 | 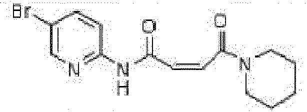 |
| 1-49 | 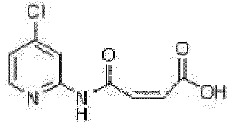 | 1-50 | 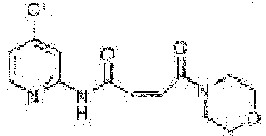 |
| 1-51 | 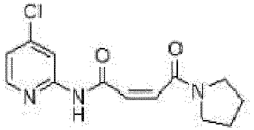 | 1-52 | 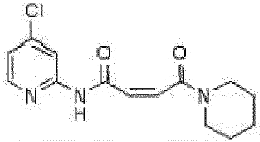 |
| 1-53 | 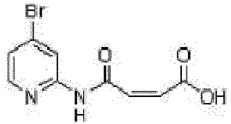 | 1-54 | 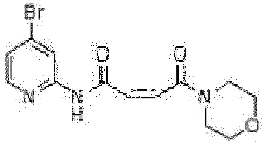 |
| 1-55 | 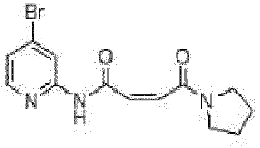 | 1-56 | 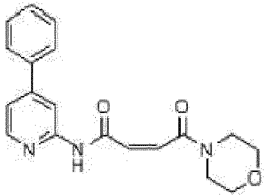 |
| 1-57 | 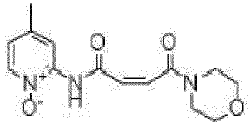 | | |

The maleic acid derivative of Chemical Fformula 1 of the present invention may be used in a form of a pharmaceutically acceptable salt. As the salt, acid addition salts which are formed from various pharmaceutically or physiologically acceptable organic or inorganic salts are useful. Examples of suitable organic acids may include carboxylic acid, phosphonic acid, sulfonic acid, acetic acid, propionic acid, octanoic acid, decanoic acid, glycolic acid, lactic acid, fumaric acid, succinic acid, adipic acid, malic acid, tartaric acid, citric acid, glutamic acid, aspartic acid, maleic acid, benzoic acid, salicylic acid, phthalic acid, phenylacetic acid, benzene sulfonic acid, 2-naphthalenesulfonic acid, methyl sulfuric acid, ethyl sulfuric acid, dodecyl sulfuric acid, and the like. Examples of suitable inorganic acids may include hydrochloric acid, sulfuric acid, or phosphoric acid.

The maleic acid derivative of Chemical Formula 1 of the present invention may include all salts, hydrates, and solvates, which may be prepared by conventional methods, as well as pharmaceutically acceptable salts thereof.

In accordance with another aspect of the present invention, there is provided a method for preparing a maleic acid derivative represented by Chemical Formula 1 above.

### <Preparation Method 1>

With respect to a preparation method of a compound of Chemical Formula 1a in which R is OH, among the derivatives of Chemical Formula 1 according to the present invention, A¹-NH₂ represented by chemical formula 2 is allowed to react with a maleic acid anhydride to prepare a maleic acid derivative in which R is OH, or a pharmaceutically acceptable salt thereof, as shown in reaction scheme 1. 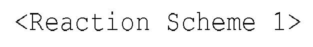 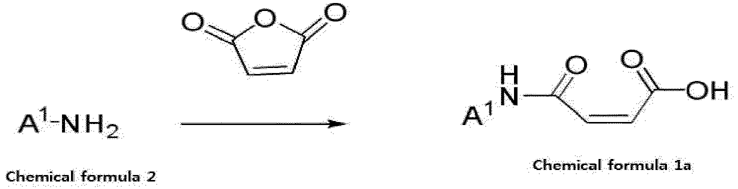 wherein in Chemical Formula 1a and Chemical Formula 2,
A¹ is heteroaryl or heterocycloalkyl containing at least one heteroatom selected from the group consisting N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group.

Hereinafter, the preparation method above will be described in detail.

The reaction between the amine of Chemical Formula 2 and the maleic acid anhydride is conducted by stirring them in a suitable solvent. Here, ether, such as diethyl ether, tetrahydrofurane, or dioxane, is preferably used as a reaction solvent, while the reaction temperature may range from room temperature to the boiling point of a reaction solvent.

### <Preparation Method 2>

A preparation method of an ester compound of Chemical Formula 1b in which R is OR¹, among the derivatives of Chemical Formula 1 according to the present invention, includes, an esterification reaction of a carboxylic acid represented by Chemical Formula 1a and alcohol R¹OH, as shown in Reaction Scheme 2. 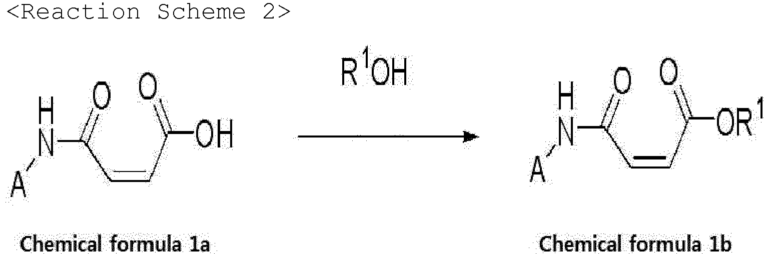 wherein in Chemical Formula 1a and Chemical Formula 1b,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
each R¹ is C₁-C₅ straight- or branched-chain alkyl, or phenyl.

Hereinafter, the preparation method above will be described in detail.

In preparation method 2, the ester compound of Chemical Formula 1b may be prepared by using an excessive amount of alcohol R¹OH and conducting the reaction in the presence of an acid catalyst. Here, the acid used as a catalyst is mainly an inorganic acid, such as hydrochloric acid or sulfuric acid, while the reaction temperature may range from room temperature to the boiling point of alcohol.

Alternatively, the ester compound of Chemical Formula 1b may be prepared by activating the carboxylic acid of Chemical Formula 1a into a mixed anhydride using an alkyl formate, or into an azide compound using diphenylphosphoryl azide, or activating the carboxylic acid of Chemical Formula 1a using a condensing agent, for example, water soluble carbodiimide (WSC) such as 1-ethyl-3-(3-dimethylaminopropyl)carbodiimide, or dicyclohexylcarbodiimide (DCC), followed by a reaction with an equivalent amount of alcohol (R¹OH). Here, an ether-based solvent such as tetrafuran, or a substituted amide-based solvent such as dimethylformamide, may be preferably used as the reaction solvent.

In addition, the reaction temperature may range from room temperature to the boiling temperature of a reaction solvent.

### <Preparation Method 3>

A preparation method of an amine compound of Chemical Formula 1c in which R is NR¹R², or a compound of Chemical Formula 1d, in which R is a cycloamine ( 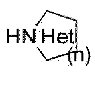 ), among the derivatives of chemical formula 1 of the present invention, includes a condensation reaction of the carboxylic acid represented by Chemical Formula 1a and an amine R¹R²NH or a cycloamine ( 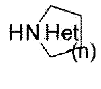 ), as shown in Reaction Scheme 3 below. 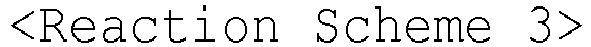 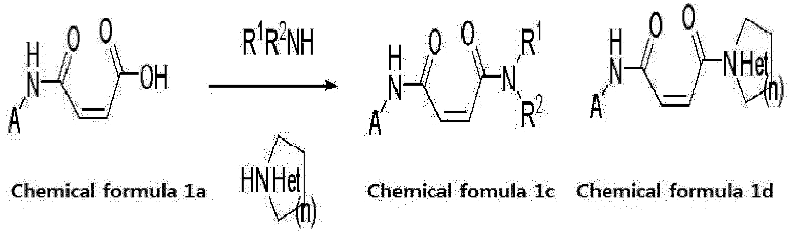 wherein in Chemical Formulas 1a, 1b, and 1c,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
R¹ and R² each are phenyl or heteroaryl having C₁-C₅ straight- or branched-chain alkyl or a substituent group; n is 0 to 2; Het is heteroaryl or heterocycloalkyl having a substituent group and containing at least one heteroatom selected from the group consisting of N, O, and S, while including N.

Hereinafter, the preparation method above will be described in detail.

In preparation method 3, the carboxylic acid compound of Chemical Formula 1a is allowed to react with an equivalent or excessive amount of amine (R¹R²NH) in the presence of a condensing agent in an appropriate reaction solvent, thereby preparing a compound of Chemical Formula 1c. The reaction temperature ranges from room temperature to the boiling point of a reaction solvent.

Herein, the condensing agent that may be used includes N,N-carbonyldiimidazole, dicyclohexylcarbodiimide (DCC), diisopropylcarbodiimide (DIPC), 1-ethyl-3-(3-dimethylaminopropyl)carbodiimide (WSC), diphenylphosphonyl azide (DPPA), or the like.

The solvent that may be used includes ether-based solvents, such as tetrahydrofurane and 1,4-dioxane, aromatic hydrocarbon solvents, such as benzene and toluene, halogenated hydrocarbon solvents, such as dichloromethane and chloroform, DMF, or mixture solvents thereof. Sodium azide (NaN₃) is allowed to react with aryl boronic acid (R⁶-B(OH)₂) in the presence of copper sulfate to prepare aryl azide (ArN₃), which is subsequently allowed to react with acetylene of Chemical Formula 4 in the presence of sodium ascorbate to prepare a compound of Chemical Formula 1c in which R³ is 1,2,3-triazole through yne-diene cyclization. Here, the solvent that may be used includes a mixture solvent of an alcohol, such as methanol or ethanol, and water, while the reaction temperature may range from room temperature to the boiling point of the solvent.

In addition, the maleic acid derivative of Chemical Formula 1 according to the present invention can be favorably used for the prevention and treatment of cancer by inhibiting the expression of AIMP2-DX2 in lung cancer cells.

The effects of the present invention are well shown in examples of the present invention.

As a result of investigating the expression level when the luciferase-AIMP2-DX2 gene-transfected lung cancer cell lines were treated with the maleic acid derivatives of Chemical Formula 1, the compounds of examples 1-1 to 1-4, 1-6, 1-12 to 1-23, 1-26, 1-29, and 1-49 to 1-56 exhibited an inhibitory effect of at least 50%, while the compounds of examples 1-14, 1-20, 1-23 and 1-49, 1-55, 1-56 exhibited an excellent inhibitory effect of at least 70%, at 5 µM concentration thereof. From the results, it was confirmed that the maleic acid derivative compounds according to the present invention inhibited the expression of AIMP2-DX2, which is known to be over-expressed in lung cancer patients [See Table 2].

In addition, a material containing, as an active ingredient, the maleic acid derivative represented by Chemical Formula 1 according to the present invention or a pharmaceutically acceptable salt thereof, suppresses the proliferation of lung cancer cells in a dose-dependent manner.

Such effects of the present invention are well shown in examples of the present invention.

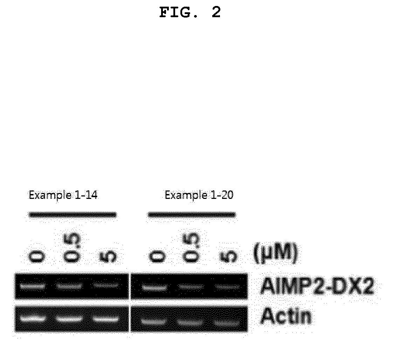

In an RT-PCR test, a material containing, as an active ingredient, the maleic acid derivative represented by Chemical Formula 1 or the pharmaceutically acceptable salt thereof inhibited mRNA of AIMP2-DX2 in a dose-dependent manner [See FIG. 2].

In addition, in the cytotoxicity test on five types of normal cell lines, the material containing, as an active ingredient, the maleic acid derivative represented by Chemical Formula 1 or the pharmaceutically acceptable salt thereof exhibited CC₅₀ values of 100 µM or more on the five types of cell lines, confirming its non-cytotoxicity [See Table 3].

Thus, it was verified that the maleic acid derivative of Chemical Formula 1 of the present invention inhibited the expression of AIMP2-DX2 in lung cancer cells, suppressed the proliferation of lung cancer cells in a dose-dependent manner, inhibited mRNA of AIMP2-DX2 in a dose-dependent manner, and was selective to lung cancer cells without cytotoxicity on normal cells.

Therefore, the present invention provides a pharmaceutical composition for preventing and treating cancer based on the inhibition of AIMP2-DX2 expression, the composition comprising, as an active ingredient, a maleic acid derivative represented by Chemical Formula 1 of the present invention or a pharmaceutically acceptable salt thereof.

Furthermore, the present invention provides a method for preventing or treating cancer, the method comprising a step of administering a maleic acid derivative represented by Chemical Formula 1 of the present invention or a pharmaceutically acceptable salt thereof to a subject in need thereof.

Furthermore, the present invention provides a use of a maleic acid derivative represented by chemical formula 1 of the present invention or a pharmaceutically acceptable salt thereof for preparing an agent for the prevention or treatment of cancer.

The cancer may include breast cancer, large intestine cancer, lung cancer, small cell lung cancer, gastric cancer, liver cancer, blood cancer, bone cancer, pancreatic cancer, skin cancer, head or neck cancer, cutaneous or intraocular melanoma, uterine cancer, ovarian cancer, rectal cancer, anal cancer, colon cancer, breast cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid carcinoma, adrenal cancer, soft tissue sarcoma, urethral cancer, penile cancer, prostate cancer, chronic or acute leukemia, lymphocyte lymphoma, bladder cancer, kidney or ureter cancer, renal cell carcinoma, renal pelvic carcinoma, CNS tumor, primary CNS lymphoma, spinal cord tumor, brain stem glioma, pituitary adenoma, and the like.

In the pharmaceutical composition according to the present invention, the maleic acid derivative of Chemical Formula 1 or the pharmaceutically acceptable salt thereof may be administered in various oral and parental dosage forms at the time of clinical administration, and may be formulated by using a diluent or an excipient, such as a filler, an extender, a binder, a wetting agent, a disintegrant, or a surfactant, which is normally used.

Solid preparations for oral administration include a tablet, a pill, a powder, granules, a capsule, a troche, and the like. These solid preparations may be prepared by mixing at least one aryl derivative of Chemical Formula 1 of the present invention or a pharmaceutically acceptable salt with at least one excipient, for example, starch, calcium carbonate, sucrose or lactose, or gelatin. In addition to the simple excipient, lubricants, such as magnesium stearate and talc, may be used. Liquid preparations for oral administration refer to a suspension, an oral solution, an emulsion, a syrup, and the like. Besides simple diluents that are frequently used, such as water and liquid paraffin, various excipients, for example, a wetting agent, a sweetener, an aroma, and a preservative may be included in the liquid preparations.

Preparations for parenteral administration include a sterilized aqueous solution, a non-aqueous solvent, a suspension solvent, an emulsion, a freeze-drying agent, and a suppository. The non-aqueous solvent and the suspension solvent may include propylene glycol, polyethylene glycol, vegetable oil such as olive oil, injectable ester such as ethylolate, and the like. As a substrate for the suppository, Witepsol, Macrogol, twin 61, cacao butter, laurin butter, glycerol, gelatin, or the like may be used.

In addition, the dose of the 1-substituted idol derivative of Chemical Formula 1 of the present invention or the pharmaceutically acceptable salt thereof to the human body may vary depending on age, body weight, and sex of a patient, the manner of administration, the health condition, and the severity of the disease. Based on the adult patient weighing 70 Kg, a dose thereof is generally 0.1-1000 mg/day, and preferably 1-500 mg/day. The derivative or the pharmaceutically acceptable salt thereof may be administered once a day or divided into multiple doses at predetermined time intervals according to the determination of his/her physician or pharmacist.

The pharmaceutical composition of the present invention may be used alone or in combination with other methods employing surgery, hormone treatment, chemical treatment, and biological response controller, for the prevention or treatment of dyslipidemia cancer.

As used herein, the term "subject" refers to an animal, preferably a mammal, and especially, an animal including a human being, and may be cells, tissues, organs, or the like, derived from the animal. The subject may be a patient in need of treatment. In addition, as used herein, the term "subject in need thereof" refers to a subject in need of the prevention or treatment of cancer.

In addition, as used herein, the term "effective amount" refers to an amount of the maleic acid derivative of Chemical Formula 1 or the pharmaceutically acceptable salt that produces an effective effect in a subject to be administered, that is, the prevention or treatment of cancer.

The maleic acid derivative of Chemical Formula 1 or the pharmaceutically acceptable salt may be administered as it is or administered in several dosage forms prepared as described above, and preferably may be administered until a desired effect, that is, the cancer prevention or treatment effect is induced. The maleic acid derivative of Chemical Formula 1 or the pharmaceutically acceptable salt thereof may be administered in various routes by methods known in the art. That is, the maleic acid derivative and the pharmaceutically acceptable salt thereof may be administered orally or parenterally, for example, by mouth, intramuscularly, intravenously, intradermally, intraarterially, intramedullarily, intrathecally, intraperitoneally, intra-nasally, intra-vaginally, intrarectally, sublingually, or subcutaneously, or may be administered through the gastrointestinal tract, mucous membranes, or respiratory tract.

Meanwhile, the maleic acid of Chemical Formula 1 or the pharmaceutically acceptable salt thereof according to the present invention can be formulated in various dosage forms depending on its desired purpose. Preparative examples for the composition of the present invention are exemplified below.

### <Preparative Example 1> Preparation of pharmaceutical preparation

**1. Preparation of powders**

| | |
|---|---|
| Compound of Chemical Formula 1 according to the present invention | 2 g |
| Lactose | 1 g |

The above ingredients were mixed, and then filled in a sealed package, thereby preparing powders.

**2. Preparation of tablets**

| | |
|---|---|
| Compound of Chemical Formula 1 according to the present invention | 100 mg |
| Corn starch | 100 mg |
| Lactose | 100 mg |
| Magnesium stearate | 2 mg |

The above ingredients were mixed, and then tablets were prepared by general tablet preparation methods.

**3. Preparation of capsules**

| | |
|---|---|
| Compound of Chemical Formula 1 according to the present invention | 100 mg |
| Corn starch | 100 mg |
| Lactose | 100 mg |
| Magnesium stearate | 2 mg |

The above ingredients were mixed, and then filled in a gelatin capsule by general capsule preparation methods, thereby preparing capsules.

**4. Preparation of pills**

| | |
|---|---|
| Compound of Chemical Formula 1 according to the present invention | 1 g |
| Lactose | 1.5 g |
| Glycerin | 1 g |
| Xylitol | 0.5 g |

The above ingredients were mixed, and then pills having 4 g per pill were prepared by conventional methods.

**5. Preparation of granules**

| | |
|---|---|
| Compound of Chemical Formula 1 according to the present invention | 150 mg |
| Soybean extract | 50 mg |
| Glucose | 200 mg |
| Starch | 600 mg |

The above ingredients were mixed, and then 100 mg of 30% ethanol was added, followed by drying at 60°C, thereby preparing granules, which were then filled in a bag.

### [Advantageous Effects]

The maleic acid derivative of Chemical Formula 1 according to the present invention has an excellent action of inhibiting AIMP2-DX2 expression and an excellent effect of suppressing the proliferation of cancer cells, and thus can be favorably used in the prevention and treatment of cancer.

### [Brief Description of the Drawings]

FIG. 1 shows results of an MTT assay conducted in order to evaluate the cytotoxic effect of the maleic acid derivatives of the present invention on lung cancer cells. The horizontal axis represents the concentration of the treated compounds.
   (Example 1-14: (*Z*)-*N*-(4-methylpyridin-2-yl)-4-oxo-4-(piperidin-1-yl)but-2-enamide, Example 1-20: *(Z)-N-(4-*methylpridin-2-yl)-4-morpholino-4-oxobut-2-enamide).
FIG. 2 shows results of an RT-PCR test to evaluate the effect of the maleic acid derivatives of the present invention on AIMP2-DX2 mRNA.
   (Example 1-14: (*Z*)-*N*-(4-methylpyridin-2-yl)-4-oxo-4-(piperidin-1-yl)but-2-enamide, Example 1-20: *(Z)-N-(4-*methylpridin-2-yl)-4-morpholino-4-oxobut-2-enamide).

### [Mode for Carrying Out the Invention]

Hereinafter, the present invention will be described in detail with reference to examples and experimental examples.

However, the following examples are merely for illustrating the present invention and are not intended to limit the scope of the present invention.

### <Example 1>

### Preparation of compounds

### <Example 1-1> Preparation of (Z)-4-oxo-4-(pyridin-2-ylamino)but-2-enoic acid

2-aminopyridine (10.6 mmol, 1.0 g) and a maleic anhydride (10.6 mmol, 1.04 g) were dissolved in diethyl ether (30 mL), followed by stirring for 5 hours. After the completion of the reaction, the precipitate was filtered, washed with hexane, and dried, to obtain the title compound (1.55 g, 76% yield) as a light orange solid.

¹H-NMR (300MHz, DMSO-d₆) δ 6.38 (brs, 1H), 6.52 (m, 2H), 6.81 (dd, *J* = 6.6, 7.8 Hz, 1H), 6.68(d, *J* = 6.6, 5.6 Hz, 1H), 7.45 (m, 1H), 7.86 (d, *J =* 5.6 Hz, 1H).

### <Example 1-2> Preparation of (Z)-4-[(4-methylpyridin-2-yl)amino]-4-oxobut-2-enoic acid

A reaction using 2-amino-4-methylpyridine as a startin material was performed by the same method as in Example 1-1 to obtain the title compound (66% yield).

¹H-NMR (300MHz, DMSO-d₆) δ 2.16 (s, 3H), 5.99 (brs, 1H), 6.37 (brs, 1H), 6.41 (d, *J* = 5.6 Hz, 1H), 6.68 (brs, 1H), 6.89 (m, 1H), 7.79 (d, *J =* 5.6 Hz, 1H).

### <Example 1-3> Preparation of (Z)-4-oxo-4-(pyridin-3-ylamino)but-2-enoic acid

A reaction using 3-aminopyridine as a starting material was performed by the same method as in Example 1-1 to obtain the title compound (80% yield).

¹H-NMR (300MHz, DMSO-d₆) δ 6.31 (d, *J* = 12.4 Hz, 1H), 6.47 (d, *J =* 12.4 Hz, 1H), 7.35 (m, 1H), 8.04 (d, *J* = 8.2 Hz, 1H), 8.27 (s, 1H), 8.73 (s, 1H).

### <Example 1-4> Preparation of (Z)-4-[(4,6-dimethylpyridin-2-yl)amino]-4-oxobut-2-enoic acid

A reaction using 2-amino-4,6-dimethylpyridine as a starting material was performed by the same method as in Example 1-1 to obtain the title compound (70% yield).

¹H-NMR (300MHz, DMSO-d₆) δ 2. 11 (s, 3H), 2.20 (s, 3H), 5.99 (brs, 1H), 6.28 (brs, 1H), 6.35 (d, *J* = 8.5 Hz, 1H), 6.83 (d, *J =* 8.5 Hz, 1H), 7.77 (s, 1H).

### <Example 1-5> Preparation of (Z)-4-[(5-chloropyridin-2-yl)amino]-4-oxobut-2-enoic acid

A reaction using 2-amino-5-chloropyridine as a starting material was performed by the same method as in Example 1-1 to obtain the title compound (68% yield).

¹H-NMR (300MHz, DMSO-d₆) δ 6.21 (brs, 1H), 6.40 (brs, 1H), 7.72 (d, *J =* 8.0 Hz, 1H), 8.03 (m, 1H), 8.30 (s, 1H).

### <Example 1-6> Preparation of (Z)-4-[(6-bromopyridin-3-yl)amino]-4-oxobut-2-en

A reaction using 3-amino-6-bromopyridine as a starting material was performed by the same method as in Example 1-1 to obtain the title compound (73% yield).

¹H-NMR (300MHz, DMSO-d₆) δ 6.21 (d, *J* = 12.0 Hz, 1H), 6.48 (d, *J* = 12.0 Hz, 1H), 7.30 (m, 1H), 8.12 (m, 1H), 8.28 (m, 1H), 8.63 (s, 1H).

### <Example 1-7> Preparation of (Z)-4-[(5-methyl-1,3,4-thiadiazol-2-yl)amino]-4-oxobut-2-enoic acid

A reaction using 2-amino-5-methyl-1,3,4-thiadiazole as a starting material was performed by the same method as in Example 1-1 to obtain the title compound (65% yield).

¹H-NMR (300MHz, DMSO-d₆) δ 2.48 (s, 3H), 6.47 (m, 2H), 7.03 (s, 1H).

### <Example 1-8> Preparation of (Z)-4-oxo-4-[(thiophen-2-ylmethyl)amino]but-2-enoic acid

A reaction using thiophen-2-yl methanamine as a starting material was performed by the same method as in Example 1-1 to obtain the title compound (81% yield).

¹H-NMR (300MHz, DMSO-d₆) δ 4.51 (m, 2H), 6.24 (m, 2H), 6.95 (dd, *J =* 5.1, 5.2 Hz, 1H), 7.01 (d, *J =* 5.2 Hz, 1H), 7.41 (d, *J =* 5.1 Hz, 1H), 9.74 (s, 1H).

### <Example 1-9> Preparation of (Z)-4-[(3-cyanothiophen-2-yl)amino]-4-oxobut-2-enoic acid

A reaction using 2-aminothiophene-3-carbonitrile as a starting material was performed by the same method as in Example 1-1 to obtain the title compound (9% yield).

¹H-NMR (DMSO-*d*₆, 300MHz) δ 6.47 (d, *J =* 12.2 Hz, 1H), 6.61 (d, *J =* 12.2 Hz, 1H), 7.20 (m, 2H), 12.01 (s, 1H).

### <Example 1-10> Preparation of (Z)-4-(isoxazol-3-ylamino)-4-oxobut-2-enoic acid

A reaction using isoxazole-3-amine as a starting material was performed by the same method as in Example 1-1 to obtain the title compound (51% yield).

¹H-NMR (300MHz, DMSO-d₆) δ 6.34 (d, *J* = 12.2 Hz, 1H), 6.53 (d, *J =* 12.2 Hz, 1H), 6.98 (d, *J* = 1.5 Hz, 1H), 8.55 (d, *J* = 1.5 Hz, 1H).

### <Example 1-11> Preparation of ethyl (Z)-4-[(3,4-dimethylphenyl)amino]-4-oxobut-2-enoate

(Z)-4-[(3,4-dimethylphenyl)amino]-4-oxobut-2-enoic acid (50 mg, 0.23 mmol) was dissolved in 5 mL of methanol, and four drops of strong hydrochloric acid were added. The mixture was stirred at room temperature for 6 hours, and an aqueous NaHCO₃ solution was added to stop the reaction, followed by extraction with CH₂Cl₂. The organic layer was dried over MgSO₄, followed by filtration, and the chemical liquid was concentrated under reduced pressure, to obtain a residue, which was then purified by silica gel column chromatography (hexane: ethyl acetate = 5: 1), thereby obtaining the title compound (48 mg, 86% yield) as a brown solid.

¹H-NMR (300 MHz, CDCl₃) δ 1.18 (t, *J* = 6.8 Hz, 3H), 2.18 (s, 3H), 2.20 (s, 3H), 4.02 (q, *J* = 6.8 Hz, 2H), 6.49 (d, *J* = 12.0 Hz, 1H), 6.94 (d, *J* = 12.0 Hz, 1H), 7.10 (d, *J* = 7.2 Hz, 1H), 7.39 (d, *J =* 7.2 Hz, 1H), 7.43 (s, 1H), 8.94 (s, 1H).

### <Example 1-12> Preparation of (Z)-4-oxo-4-(piperidin-1-yl)-N-(m-tolyl)but-2-enamide

(*Z*)-4-oxo-4-(*m*-tolylamino)but-2-enoic acid (0.05 g, 0.24 mmol) was dissolved in anhydrous THF (1.5 mL), and Et₃N (0.04 mL, 0.29 mmol) was added, followed by stirring at room temperature for 10 minutes. Methyl chloroformate (0.025 ml, 0.25 mmol) was added to the reaction mixture, followed by stirring for 30 minutes, and then piperidine (0.02 mL, 0.24 mmol) was added, followed by stirring for 18 hours. All volatiles were removed under reduced pressure. The residue was fractionated with CH₂Cl₂ and water, and the organic layer was dried over Na₂SO₄, followed by filtration. The filtrate was concentrated under reduced pressure. The residue was purified by silica gel column chromatography (hexane: ethyl acetate = 3:1 to 1:2) to obtain the title compound (51 mg, 75% yield) as an ivory solid.

¹H-NMR (300MHz, CDCl₃) δ 1.52~1.64 (m, 6H), 2.34 (s, 3H), 3.46 (m, 2H), 3.63 (m, 2H), 6.21 (d, *J* = 13.2 Hz, 1H), 6.46 (d, *J =* 13.2 Hz, 1H), 6.92 (d, *J =* 7.4 Hz, 1H), 7.20 (dd, *J* = 7.4, 8.0 Hz, 1H), 7.44 (m, 1H), 7.45 (m, 1H), 10.48 (s, 1H).

### <Example 1-13> Preparation of (Z)-N-(3,5-dimethylphenyl)-4-oxo-4-(piperidin-1-yl)but-2-enamide

A reaction using (*Z*)-4-[(2,5-dimethylphenyl)amino]-4-oxobut-2-enoic acid as a starting material was performed by the same method as in Example 1-12 to obtain the title compound (92% yield) as a pale yellow solid.

¹H-NMR (300MHz, CDCl₃) δ 1.46~1.60 (m, 6H), 2.29 (s, 6H), 3.45 (m, 2H), 3.64 (m, 2H), 6.20 (d, *J* = 13.0 Hz, 1H), 6.45 (d, *J* = 13.0 Hz, 1H), 6.74 (m, 1H), 7.27 (m, 2H), 10.34 (s, 1H).

### <Example 1-14> Preparation of (Z)-N-(4-methylpyridin-2-yl)-4-oxo-4-(piperidin-1-yl)but-2-enamide

A reaction using the compound of Example 1-2 as a starting material was performed by the same method as in Example 1-12 to obtain the title compound (63% yield) as a brown solid.

¹H-NMR (300MHz, CDCl₃) δ 1.42~1.71 (m, 6H), 2.36 (s, 3H), 3.40 (m, 2H), 3.48 (m, 2H), 6.82 (m, 2H), 7.82 (s, 1H), 7.93 (s, 1H), 8.11 (d, *J* = 5.5 Hz, 1H), 8.21 (d, *J* = 5.5 Hz, 1H).

### <Example 1-15> Preparation of (Z)-4-oxo-4-(piperidin-1-yl)-N-(pyridin-2-yl)but-2-enamide

A reaction using the compound of Example 1-1 as a starting material was performed by the same method as in Example 1-12 to obtain the title compound (12% yield) as a brown solid.

¹H-NMR (300MHz, CDCl₃) δ 1.54~1.70 (m, 6H), 3.53 (m, 4H), 6.50 (d, *J* = 8.5 Hz, 1H), 6.64 (m, 1H), 6.99 (m, 1H), 7.43 (m, 1H), 7.97 (d, *J =* 8.5 Hz, 1H), 8.87 (d, *J* = 4.8 Hz, 1H).

### <Example 1-16> Preparation of (Z)-4-oxo-4-(piperidin-1-yl)-N-(pyridin-3-yl)but-2-enamide

A reaction using the compound of Example 1-3 as a starting material was performed by the same method as in Example 1-12 to obtain the title compound (91% yield) as a brown solid.

¹H-NMR (300MHz, CDCl₃) δ 1.44~1.54 (m, 6H), 2.84 (m, 2H), 2.89 (m, 2H), 6.25 (d, *J =* 13.4 Hz, 1H), 6.53 (d, *J* = 13.7 Hz, 1H), 7.24 (dd, *J =* 4.7, 8.3 Hz, 1H), 8.20 (d, *J =* 8.3 Hz, 1H), 8.33 (d, *J =* 4.7 Hz, 1H), 8.71 (s, 1H), 11.12 (s, 1H).

### <Example 1-17> Preparation of (Z)-N-(4,6-dimethylpyridin-2-yl)-4-oxo-4-(piperidin-1-yl)but-2-enamide

A reaction using the compound of Example 1-4 as a starting material was performed by the same method as in Example 1-12 to obtain the title compound (41% yield) as a brown solid.

¹H-NMR (300MHz, CDCl₃) δ 1.50~1.59 (m, 6H), 2.20 (s, 3H), 2.35 (s, 3H), 3.41 (m, 4H), 6.11 (d, *J* = 12.1 Hz, 1H), 6.51 (d, *J* = 12.1 Hz, 1H), 6.73(s, 1H), 7.89 (s, 1H), 9.25 (s, 1H).

### <Example 1-18> Preparation of (Z)-4-morpholino-4-oxo-N-(m-tolyl)but-2-enamide

(*Z*)-4-oxo-4-(*m*-tolylamino)but-2-enoic acid (0.1 g, 0.49 mmol) was dissolved in anhydrous THF (5 mL), and Et₃N (0.09 mL, 0.59 mmol) was added, followed by stirring at room temperature for 10 minutes. Methyl chloroformate (0.025 ml, 0.25 mmol) was added to the reaction mixture, followed by stirring for 30 minutes, and then morpholine (0.04 mL, 0.49 mmol) was added, followed by stirring for 18 hours. All volatiles were removed under reduced pressure. The residue was fractionated with CH₂Cl₂ and water, and the organic layer was dried over Na₂SO₄, followed by filtration. The filtrate was concentrated under reduced pressure. The residue was purified by silica gel column chromatography (hexane: ethyl acetate = 3:1 to 1:2) to obtain the title compound (129 mg, 97% yield) as a white solid.

¹H-NMR (300MHz, CDCl₃) δ 2.33 (s, 3H), 3.72 (m, 8H), 6.25 (d, *J* = 12.6 Hz, 1H), 6.39 (d, *J* = 12.6 Hz, 1H), 6.93 (dd, *J =* 6.6, 8.5 Hz, 1H), 7.28 (d, *J =* 6.6 Hz, 1H), 7.39 (d, *J =* 8.5 Hz, 1H), 7.44 (s, 1H), 9.96 (s, 1H).

### <Example 1-19> Preparation of (Z)-N-(3,5-dimethylphenyl)-4-morpholino-4-oxobut-2-enamide

A reaction using (*Z*)-4-[(2,5-dimethylphenyl)amino]-4-oxobut-2-enoic acid as a starting material was performed by the same method as in Example 1-18 to obtain the title compound (92% yield) as a white solid.

¹H-NMR (300MHz, CDCl₃) δ 2. 29 (s, 6H), 3.72 (m, 8H), 6.25 (d, *J* = 12.8 Hz, 1H), 6.40 (d, *J* = 12.8 Hz, 1H), 7.17 (s, 1H), 7.35 (s, 1H), 7.4 6(s, 1H), 9.85 (s, 1H).

### <Example 1-20> Preparation of (Z)-N-(4-methylpyridin-2-yl)-4-morpholino-4-oxobut-2-enamide

A reaction using the compound of Example 1-2 as a starting material was performed by the same method as in Example 1-18 to obtain the title compound (46% yield) as a brown solid.

¹H-NMR (300MHz, CDCl₃) δ 2.36 (s, 3H), 3.81 (m, 8H), 6.82 (m, 2H), 7.27 (m, 2H), 8.15 (d, *J* = 5.1 Hz, 1H), 9.07 (s, 1H).

### <Example 1-21> Preparation of (Z)-4-oxo-4-(pyrrolidin-1-yl)-N-(m-tolyl)but-2-enamide

(*Z*)-4-oxo-4-(*m*-tolylamino)but-2-enoic acid (0.1 g, 0.49 mmol) was dissolved in anhydrous THF (5 mL), and Et₃N (0.09 mL, 0.59 mmol) was added, followed by stirring at room temperature for 10 minutes. Methyl chloroformate (0.025 ml, 0.25 mmol) was added to the reaction liquid, followed by stirring for 30 minutes, and then pyrrolidine (0.04 mL, 0.49 mmol) was added, followed by stirring for 18 hours. All volatiles were removed under reduced pressure. The residue was fractionated with CH₂Cl₂ and water, and the organic layer was dried over Na₂SO₄, followed by filtration. The filtrate was concentrated under reduced pressure. The residue was purified by silica gel column chromatography (hexane: ethyl acetate = 5:1 to 1:5) to obtain the title compound (74 mg, 59% yield) as a pale yellow solid.

¹H-NMR (300MHz, CDCl₃) δ 1.90∼2.06 (m, 4H), 2.34 (s, 3H), 3.50∼3.62 (m, 4H), 6.26 (d, *J =* 13.2 Hz, 1H), 6.38 (d, *J =* 13.2 Hz, 1H), 6.90 (d, *J =* 7.8 Hz, 1H), 7.20 (dd, *J =* 5.8, 7.8 Hz, 1H), 7.26 (s, 1H), 7.50 (d, *J =* 5.8 Hz, 1H), 12.08 (s, 1H).

### <Example 1-22> Preparation of (Z)-N-(3,5-dimethylphenyl)-4-oxo-4-(pyrrolidin-1-yl)but-2-enamide

A reaction using (*Z*)-4-[(2,5-dimethylphenyl)amino]-4-oxobut-2-enoic acid as a starting material was performed by the same method as in Example 1-21 to obtain the title compound (49% yield) as a yellow solid.

¹H-NMR (300MHz, CDCl₃) δ 1.93∼2.05 (m, 4H), 2.30 (s, 6H), 3.51∼3.62 (m, 4H), 6.26 (d, *J* = 13.6 Hz, 1H), 6.37 (d, *J* = 13.6 Hz, 1H), 6.74 (s, 1H), 7.33 (s, 1H), 7.37 (s, 1H), 11.96 (s, 1H).

### <Example 1-23> Preparation of (Z)-N-(4-methylpyridin-2-yl)-4-oxo-4-(pyrrolidin-1-yl)but-2-enamide

A reaction using the compound of Example 1-2 as a starting material was performed by the same method as in Example 1-21 to obtain the title compound (34% yield) as a brown solid.

¹H-NMR (300MHz, CDCl₃) δ 1.76∼2.11 (m, 4H), 3.29∼3.63 (m, 4H), 3.81 (s, 3H), 6.96 (d, *J* = 13.6 Hz, 1H), 7.11 (d, *J =* 13.6 Hz, 1H), 7.87 (s, 1H), 8.01 (d, *J* = 2.6 Hz, 1H), 8.16 (d, *J =* 2.6 Hz, 1H), 9.16 (s, 1H).

### <Example 1-24> Preparation of (Z)-N-(pyridin-2-yl)-4-oxo-4-(pyrrolidin-1-yl)but-2-enamide

A reaction using the compound of Example 1-1 as a starting material was performed by the same method as in Example 1-21 to obtain the title compound (11% yield) as a brown solid.

¹H-NMR (300MHz, CDCl₃) δ 1.57∼1.81 (m, 4H), 3.29∼3.71 (m, 4H), 6.99 (m, 2H), 7.69 (m, 1H), 8.00 (m, 2H), 8.27 (m, 1H), 8.37 (d, *J =* 4.8 Hz, 1H).

### <Example 1-25> Preparation of (Z)-N-(4-bromophenyl)-4-oxo-4-(piperidin-1-yl)but-2-enamide

A reaction using (Z)-4-[(4-bromophenyl)amino]-4-oxobut-2-enoic acid as a starting material was performed by the same method as in Example 1-12 to obtain the title compound (99% yield) as a white solid.

¹H-NMR (300MHz, CDCl₃) δ 1.54∼1.63 (m, 6H), 3.45 (m, 2H), 3.63(m, 2H), 6.21 (d, *J =* 13.1 1 Hz, 1H), 6.45 (d, *J* = 13.1 Hz, 1H), 7.39 (d, *J* = 8.8 Hz, 2H), 7.53 (d, *J* = 8.8 Hz, 2H), 10.82 (s, 1H).

### <Example 1-26> Preparation of (Z)-N-(4-chlorophenyl)-4-oxo-4-(piperidin-1-yl)but-2-enamide

A reaction using (*Z*)-4-[(4-chlorophenyl)amino]-4-oxobut-2-enoic acid as a starting material was performed by the same method as in Example 1-12 to obtain the title compound (99% yield) as a white solid.

¹H-NMR (300MHz, CDCl₃) δ 1.56∼1.67 (m, 6H), 3.46 (m, 2H), 3.64(m, 2H), 6.22 (d, *J* = 13.1 Hz, 1H), 6.45 (d, *J* = 13.1 Hz, 1H), 7.24 (d, *J* = 8.9 Hz, 2H), 7.58 (d, *J* = 8.9 Hz, 2H), 10.80 (s, 1H).

### <Example 1-27> Preparation of (Z)-N-(4-bromophenyl)-4-morpholino-4-oxobut-2-enamide

A reaction using (*Z*)-4-[(4-bromophenyl)amino]-4-oxobut-2-enoic acid as a starting material was performed by the same method as in Example 1-18 to obtain the title compound (42% yield) as a brown solid.

¹H-NMR (300MHz, CDCl₃) δ 3.69 (m, 8H), 6.26 (d, *J =* 13.3 Hz, 1H), 6.45 (d, *J* = 13.3 Hz, 1H), 7.43 (d, *J =* 8.9 Hz, 2H), 7.54 (d, *J =* 8.9 Hz, 2H), 10.59 (s, 1H).

### <Example 1-28> Preparation of (Z)-N-(5-chloropyridin-2-yl)-4-(piperidin-1-yl)-4-oxobut-2-enamide

A reaction using the compound of Example 1-5 as a starting material was performed by the same method as in Example 1-12 to obtain the title compound (70% yield) as a brown solid.

¹H-NMR (300MHz, CDCl₃) δ 1.59∼1.66 (m, 6H), 3.38∼3.48 (m, 4H), 6.21 (d, *J =* 12.9 Hz, 1H), 6.56 (d, *J* = 12.9 Hz, 1H), 8.01 (d, *J* = 5.7 Hz, 1H), 8.24 (m, 1H), 8.29 (d, *J =* 2.6 Hz, 1H), 10.85 (s, 1H).

### <Example 1-29> Preparation of (Z)-N-(6-bromopyridin-3-yl)-4-(piperidin-1-yl)-4-oxobut-2-enamide

A reaction using the compound of Example 1-6 as a starting material was performed by the same method as in Example 1-12 to obtain the title compound (84% yield) as a brown solid.

¹H-NMR (300MHz, CDCl₃) δ 1.62∼1.66 (m, 6H), 3.47 (m, 2H), 3.59 (m, 2H), 6.25 (d, *J =* 12.9 Hz, 1H), 6.52 (d, *J* = 12.9 Hz, 1H), 7.59 (m, 1H), 8.47(m, 1H), 8.55 (d, *J* = 2.5 Hz, 1H), 10.13 (s, 1H).

### <Example 1-30> Preparation of (Z)-N-(4-chlorophenyl)-4-oxo-4-(pyrrolidin-1-yl)but-2-enamide

A reaction using (*Z*)-4-[(4-chlorophenyl)amino]-4-oxobut-2-enoic acid as a starting material was performed by the same method as in Example 1-21 to obtain the title compound (29% yield) as a brown solid.

¹H-NMR (300MHz, CDCl₃) δ 1.89∼2.08 (m, 4H), 3.56 (m, 2H), 3.67(m, 2H), 7.30 (d, *J =* 8.9 Hz, 2H), 7.40 (d, *J* = 8.9 Hz, 2H), 9.57 (s, 1H).

### <Example 1-31> Preparation of (Z)-N-(4-chlorophenyl)-4-morpholino-4-oxobut-2-enamide

A reaction using (*Z*)-4-[(4-chlorophenyl)amino]-4-oxobut-2-enoic acid as a starting material was performed by the same method as in Example 1-18 to obtain the title compound (41% yield) as a brown solid.

¹H-NMR (300MHz, CDCl₃) δ 3.70 (m, 8H), 6.26 (d, *J =* 12.8 Hz, 1H), 6.40 (d, *J* = 12.8 Hz, 1H), 7.25 (d, *J* = 9.0 Hz, 2H), 7.57 (d, *J =* 9.0 Hz, 2H), 10.45 (s, 1H).

### <Example 1-32> Preparation of (Z)-N-(6-bromopyridin-3-yl)-4-morpholino-4-oxobut-2-enamide

A reaction using the compound of Example 1-6 as a starting material was performed by the same method as in Example 1-18 to obtain the title compound (73% yield) as a pale red solid.

¹H-NMR (300MHz, CDCl₃) δ 3.74 (m, 8H), 7.50∼7.55 (m, 2H), 7.59(m, 1H), 8.43 (m, 1H), 8.47 (d, *J =* 2.9 Hz, 1H), 8.60 (s, 1H).

### <Example 1-33> Preparation of (Z)-N-(5-chloropyridin-2-yl)-4-morpholino-4-oxobut-2-enamide

A reaction using the compound of Example 1-5 as a starting material was performed by the same method as in Example 1-18 to obtain the title compound (76% yield) as a brown solid.

¹H-NMR (300MHz, CDCl₃) δ 3. 81 (m, 8H), 7.39 (d, *J* = 12.7 Hz, 1H), 7.53 (d, *J* = 12.7 Hz, 1H), 7.96 (d, *J =* 9.1 Hz, 1H), 8.26 (d, *J* = 9.1 Hz, 1H), 8.31 (s, 1H), 8.86 (s, 1H).

### <Example 1-34> Preparation of (Z)-N-(5-chloropyridin-2-yl)-4-oxo-4-(pyrrolidin-1-yl)but-2-enamide

A reaction using the compound of Example 1-5 as a starting material was performed by the same method as in Example 1-21 to obtain the title compound (81% yield) as a brown solid.

¹H-NMR (300MHz, CDCl₃) δ 1.89∼2.08 (m, 4H), 3.49 (m, 2H), 3.68 (m, 2H), 7.27∼7.40 (m, 2H), 7.66 (d, *J* = 9.6 Hz, 1H), 7.96 (d, *J =* 9.6 Hz, 1H), 8.23 (m, 1H), 9.36 (s, 1H).

### <Example 1-35> Preparation of (Z)-N-(6-bromopyridin-3-yl)-4-oxo-4-(pyrrolidin-1-yl)but-2-enamide

A reaction using the compound of Example 1-6 as a starting material was performed by the same method as in Example 1-21 to obtain the title compound (27% yield) as a yellow solid.

¹H-NMR (300MHz, CDCl₃) δ 1.89∼2.08 (m, 4H), 3.59 (m, 2H), 3.70(m, 2H), 7.35∼7.54 (m, 3H), 8.33 (d, *J* = 8.9 Hz, 1H), 8.59 (s, 1H), 10.12 (s, 1H).

### <Example 1-36> Preparation of (Z)-N-(5-methyl-1,3,4-thiadiazol-2-yl)-4-oxo-4-(pyrrolidin-1-yl)but-2-enamide

A reaction using the compound of Example 1-7 as a starting material was performed by the same method as in Example 1-21 to obtain the title compound (5% yield) as a white solid.

¹H-NMR (300MHz, CDCl₃) δ 1.89∼2.02 (m, 4H), 2.70 (s, 3H), 3.59 (m,2H), 4.14 (m, 2H), 6.87 (d, *J* = 15.3 Hz, 1H), 7.27 (d, *J =* 15.3 Hz, 1H), 11.68 (s, 1H).

### <Example 1-37> Preparation of (Z)-4-oxo-4-(pyrrolidin-1-yl)-N-(thiophen-2-ylmethyl)but-2-enamide

A reaction using the compound of Example 1-8 as a starting material was performed by the same method as in Example 1-21 to obtain the title compound (83% yield) as an ivory solid.

¹H-NMR (300MHz, CDCl₃) δ 1.79∼2.00 (m, 4H), 3.31 (m, 2H), 3.59 (m, 2H), 4.71 (m, 2H), 6.93∼6.99 (m, 2H), 7.19∼7.28 (m, 3H), 7.65 (s, 1H).

### <Example 1-38> Preparation of (Z)-4-oxo-4-(piperidin-1-yl)-N-(thiophen-2-ylmethyl)but-2-enamide

A reaction using the compound of Example 1-8 as a starting material was performed by the same method as in Example 1-12 to obtain the title compound (39% yield) as an ivory solid.

¹H-NMR (300MHz, CDCl₃) δ 1.47∼1.65 (m, 6H), 3.39 (m, 2H), 3.53 (m, 2H), 4.63 (m, 2H), 6.09 (d, *J* = 12.7 Hz, 1H), 6.40 (d, *J* = 12.7 Hz, 1H), 6.93 (dd, *J* = 3.2, 5.3 Hz, 1H), 6.99 (d, *J* = 3.2 Hz, 1H), 7.19 (d, *J* = 5.3 Hz, 1H), 8.26 (s, 1H).

### <Example 1-39> Preparation of (Z)-4-morpholino-4-oxo-N-(thiophen-2-ylmethyl)but-2-enamide

A reaction using the compound of Example 1-8 as a starting material was performed by the same method as in Example 1-18 to obtain the title compound (23% yield) as an ivory solid.

¹H-NMR (300MHz, CDCl₃) δ 3.54∼3.71 (m, 8H), 4.72 (m, 2H), 6.82∼ 6.95 (m, 2H), 6.97 (d, *J =* 1.0 Hz, 1H), 6.99 (dd, *J* = 1.0, 5.5 Hz, 1H), 7.24 (d, *J* = 5.5 Hz, 1H), 7.41 (brs, 1H).

### <Example 1-40> Preparation of (Z)-N-(3-cyanothiophen-2-yl)-4-oxo-4-(pyrrolidin-1-yl)but-2-enamide

A reaction using the compound of Example 1-9 as a starting material was performed by the same method as in Example 1-21 to obtain the title compound (9% yield) as a light green solid.

¹H-NMR (300MHz, CDCl₃) δ 1.91∼2.07 (m, 4H), 3.57∼3.71 (m, 4H), 6.38 (d, *J* = 13.5 Hz, 1H), 6.56 (d, *J* = 13.5 Hz, 1H), 6.85 (d, *J* = 5.9 Hz, 1H), 7.02 (d, *J* = 5.9 Hz, 1H), 11.02 (s, 1H).

### <Example 1-41> Preparation of (Z)-N-(3-cyanothiophen-2-yl)-4-morpholino-4-oxobut-2-enamide

A reaction using the compound of Example 1-9 as a starting material was performed by the same method as in Example 1-18 to obtain the title compound (32% yield) as a pale yellow solid.

¹H-NMR (300MHz, CDCl₃) δ 3.61∼3.82 (m, 8H), 6.93 (d, *J* = 5.6 Hz, 1H), 7.02 (d, *J* = 5.6 Hz, 1H), 7.49 (d, *J* = 15.3 Hz, 1H), 7.60 (d, *J =* 15.3 Hz, 1H), 10.41 (s, 1H).

### <Example 1-42> Preparation of (Z)-N-(3-cyanothiophen-2-yl)-4-oxo-4-(piperidin-1-yl)but-2-enamide

A reaction using the compound of Example 1-9 as a starting material was performed by the same method as in Example 1-12 to obtain the title compound (61% yield) as an ivory solid.

¹H-NMR (300MHz, CDCl₃) δ 1.57∼1.69 (m, 6H), 3.55 (m, 2H), 3.69 (m, 2H), 6.91 (d, *J =* 5.8 Hz, 1H), 7.01 (d, *J* = 5.8 Hz, 1H), 7.38 (d, *J* = 15.1 Hz, 1H), 7.64 (d, *J =* 15.1 Hz, 1H), 10.54 (s, 1H).

### <Example 1-43> Preparation of (Z)-N-(3-cyanothiophen-2-yl)-4-oxo-4-(1H-pyrazol-1-yl)but-2-enamide

The compound of Example 1-9 (0.1 g, 0.49 mmol) was dissolved in anhydrous THF (5 mL), and Et₃N (0.09 mL, 0.59 mmol) was added, followed by stirring at room temperature for 10 minutes. Methyl chloroformate (0.025 ml, 0.25 mmol) was added to the reaction liquid, followed by stirring for 30 minutes, and then pyrazole (0.04 mL, 0.55 mmol) was added, followed by stirring for 18 hours. All volatiles were removed under reduced pressure. The residue was fractionated with CH₂Cl₂ and water, and the organic layer was dried over Na₂SO₄, followed by filtration. The filtrate was concentrated under reduced pressure. The residue was purified by silica gel column chromatography (hexane: ethyl acetate = 3:1) to obtain the title compound (40 mg, 32% yield) as a white solid.

¹H-NMR (300MHz, CDCl₃) δ 6.91∼7.04 (m, 3H), 7.34∼7.36 (m, 3H), 8.15 (m, 1H), 10.48 (s, 1H).

### <Example 1-44> Preparation of (Z)-N-(isoxazol-3-yl)-4-oxo-4-(1H-pyrazol-1-yl)but-2-enamide

A reaction using the compound of Example 1-10 as a starting material was performed by the same method as in Example 1-43 to obtain the title compound (24% yield) as a white solid.

¹H-NMR (300MHz, CDCl₃) δ 6.33 (d, 1H, *J* = 12.9 Hz), 6.46 (d, 1H, *J =* 12.9 8Hz), 6.90 (m, 1H), 7.01 (d, 1H, *J* = 1.7 Hz), 7.52 (m, 1H), 8.14 (m, 1H), 8.31 (d, 1H, *J* = 1.7 Hz), 11.38 (s, 1H).

### <Example 1-45> Preparation of (Z)-4-[(5-bromopyridin-2-yl)amino]-4-oxobut-2-enoic acid

A reaction using 2-amino-5-bromopyridine as a starting material was performed by the same method as in Example 1-1 to obtain the title compound (59% yield).

¹H-NMR (300MHz, CDCl₃) δ 6.19 (s, 1H), 7.51 (d, 1H, *J* = 8.8 Hz), 7.93 (d, 1H, *J* = 8.8 Hz), 8.01 (d, 1H, *J =* 8.9 Hz), 8.08 (d, 1H, *J* = 8.9 Hz), 8.44 (s, 1H), 11.01 (s, 1H).

### <Example 1-46> Preparation of (Z)-N-(5-bromopyridin-2-yl)-4-morpholino-4-oxobut-2-enamide

A reaction using the compound of Example 1-45 as a starting material was performed by the same method as in Example 1-18 to obtain the title compound (53% yield).

¹H-NMR (300MHz, CDCl₃) δ 3.84 (m, 8H), 7.79 (m, 2H), 7.96 (m, 2H), 8.34 (s, 1H), 8.95 (s, 1H).

### <Example 1-47> Preparation of (Z)-N-(5-bromopyridin-2-yl)-4-oxo-4-(pyrrolidin-1-yl)but-2-enamide

A reaction using the compound of Example 1-45 as a starting material was performed by the same method as in Example 1-21 to obtain the title compound (62% yield).

¹H-NMR (300MHz, CDCl₃) δ 1.85∼1.95 (m, 4H), 3.44∼3.52 (m, 4H), 6.80 (m, 2H), 7.60 (m, 2H), 8.25 (s, 1H), 8.62 (s, 1H).

### <Example 1-48> Preparation of (Z)-N-(5-bromopyridin-2-yl)-4-oxo-4-(piperidin-1-yl)but-2-enamide

A reaction the compound of Example 1-45 as a starting material was performed by the same method as in Example 1-12 to obtain the title compound (54% yield).

¹H-NMR (300MHz, CDCl₃) δ 1.60∼1.67 (m, 6H), 3.56∼3.67 (m, 4H), 6.42 (d, 1H, *J =* 8.9 Hz), 6.75 (d, 1H, *J =* 15.1 Hz), 7.59 (d, 1H, *J =* 15.1 Hz), 7.94 (d, 1H, *J* = 8.9 Hz), 8.10 (s, 1H), 9.56 (s, 1H).

### <Example 1-49> Preparation of (Z)-4-[(4-chloropyridin-2-yl)amino]-4-oxobut-2-enoic acid

A reaction using 2-amino-4-chloropyridine as a starting material was performed by the same method as in Example 1-1 to obtain the title compound (65% yield).

¹H-NMR (300MHz, CDCl₃) δ 6.58 (d, 1H, *J* = 5.3 Hz), 7.27 (d, 1H, *J =* 5.3 Hz), 7.86 (d, 1H, *J* = 5.5 Hz), 8.18 (s, 1H), 8.33 (d, 1H, *J* = 5.5 Hz), 11.13 (s, 1H).

### <Example 1-50> Preparation of (Z)-N-(4-chloropyridin-2-yl)-4-morpholino-4-oxobut-2-enamide

A reaction using the compound of Example 1-49 as a starting material was performed by the same method as in Example 1-18 to obtain the title compound (68% yield).

¹H-NMR (300MHz, CDCl₃) δ 3.72 (m, 8H), 7.01 (d, 1H, *J* = 5.2 Hz), 7.09 (d, 1H, *J* = 5.2 Hz), 8.09 (s, 1H), 8.17 (d, 1H, *J =* 5.3 Hz), 8.22 (d, 1H, *J* = 5.3 Hz), 8.60 (s, 1H).

### <Example 1-51> Preparation ofZ)-N-(4-chloropyridin-2-yl)-4-oxo-4-(pyrrolidin-1-yl)but-2-enamide

A reaction using the compound of Example 1-49 as a starting material was performed by the same method as in Example 1-21 to obtain the title compound (58% yield).

¹H-NMR (300MHz, CDCl₃) δ 1.91∼1.99 (m, 4H), 3.61∼3.67 (m, 4H), 7.22 (d, 1H, *J =* 5.4 Hz), 7.41 (d, 1H, *J* = 5.4 Hz), 8.08 (s, 1H), 8.17 (d, 1H, *J* = 5.4 Hz), 8.27 (d, 1H, *J =* 5.4 Hz), 8.51 (s, 1H).

### <Example 1-52> Preparation of (Z)-N-(4-chloropyridin-2-yl)-4-oxo-4-(piperidin-1-yl)but-2-enamide

A reaction using the compound of Example 1-49 as a starting material was performed by the same method as in Example 1-12 to obtain the title compound (54% yield).

¹H-NMR (300MHz, CDCl₃) δ 1.60∼1.67 (m, 6H), 3.56∼3.67 (m, 4H), 7.11 (d, 1H, *J =* 5.2 Hz), 7.28 (d, 1H, *J* = 5.2 Hz), 8.08 (s, 1H), 8.18 (d, 1H, *J* = 5.3 Hz), 8.25 (d, 1H, *J =* 5.3 Hz), 8.58 (s, 1H).

### <Example 1-53> Preparation of (Z)-4-[(4-bromopyridin-2-yl)amino]-4-oxobut-2-enoic acid

A reaction using 4-bromo-2-aminopyridine as a starting material was performed by the same method as in Example 1-1 to obtain the title compound (65% yield) as a green solid.

¹H-NMR (300MHz, DMSO-*_{d6}*) δ 6.14 (s, 1H), 7.40 (d, *J* = 7.0 Hz, 1H), 7.80 (d, *J* = 6.2 Hz, 2H), 8.26 (d, *J* = 7.0 Hz, 1H), 8.35 (s, 1H), 11.12 (s, 1H).

### <Example 1-54> Preparation of (Z)-N-(4-bromopyridin-2-yl)-4-morpholino-4-oxobut-2-enamide

A reaction using the compound of Example 1-53 as a starting material was performed by the same method as in Example 1-18 to obtain the title compound (26% yield) as a white solid.

¹H-NMR (300MHz, CDCl₃) δ 3.67□3.74 (m, 6H), 3.92 (t, *J* = 3.8 Hz, 2H), 7.24 (d, *J* = 5.0 Hz, 1H), 7.53 (d, *J =* 7.0 Hz, 2H), 8.10 (d, *J* = 5.0 Hz, 1H), 8.59 (s, 1H), 9.53 (s, 1H).

### <Example 1-55> Preparation of (Z)-N-(4-bromopyridin-2-yl)-4-oxo-4-(pyrrolidin-1-yl)but-2-enamide

A reaction using the compound of Example 1-53 as a starting material was performed by the same method as in Example 1-21 to obtain the title compound (92% yield) as a white solid.

¹H-NMR (300MHz, CDCl₃) δ 1.97□2.21 (m, 4H), 3.64□3.80 (m, 4H), 6.78 (d, *J =* 5.1 Hz, 1H), 7.73 (d, *J* = 6.5 Hz, 2H), 8.06 (d, *J* = 5.1 Hz, 1H), 8.44 (s, 1H), 9.03 (s, 1H).

### <Example 1-56> Preparation of (Z)-4-morpholino-4-oxo-N-(4-phenylpyridin-2-yl)-but-2-enamide

The compound of Example 1-54 (32 mg, 0.09 mmol), phenyl boronic acid (10 mg, 0.11 mmol), Pd(dppf)Cl₂ (4 mg, 0.0035 mmol), and Na₂CO₃ (20 mg, 0.18 mmol) were dissolved in a mixture of ether/water (2:1), followed by stirring 80°C for 6 hours. 1 N HCl liquid was added to stop the reaction, and then was filtered by passing through a celite pad, and the filtrate was concentrated under reduced pressure. The residue was purified by silica gel column chromatography (hexane: ethyl acetate = 3:1) to obtain the title compound (11 mg, 36% yield).

¹H-NMR (300MHz, CDCl₃) δ 3.64∼3.75 (m, 6H), 6.81 (d, *J =* 5.2 Hz, 1H), 7.20 (d, *J =* 5.2 Hz, 1H), 7.40∼7.56 (m, 3H), 7.78 (d, *J* = 7.3 Hz, 2H), 7.94 (s, 1H), 8.18 (m, 2H), 10.51 (s, 1H).

### <Example 1-57> Preparation of (Z)-4-methyl-2-(4-morpholino-4-oxo-but-2-enamido)pyridine 1-oxide

(Z)-4-morpholino-4-oxobut-2-enoic acid (50 mg, 0.40 mmol), benzotriazol-1-yloxy-tris(dimethylamino)-phosphonium chloride (212 mg, 0.48 mmol), and diethylpropyl amine (0.173 mL, 1.0 mmol) were dissolved in DMF (1 mL), followed by stirring at room temperature for 5 minutes, and then 2-amino-4-methylpyridine-1-oxide (55 mg, 0.44 mmol) was added, followed by stirring at room temperature for 1 hour. The volatile materials were removed under reduced pressure, and then the residue was purified by silica gel column chromatography (5-15% methanol in CH₂Cl₂) to obtain the title compound (90 mg, 77% yield).

¹H-NMR (300MHz, CDCl₃) δ 2.45 (s, 3H), 3.68 (m, 8H), 6. 82 (d, *J* = 5.2 Hz, 1H), 7.08 (d, *J* = 5.2 Hz, 1H), 7. 21 (d, *J* = 7.0 Hz, 1H), 7.85 (d, *J* = 7.0 Hz, 1H), 10.87 (s, 1H).

### <Example 2>

### Evaluation on the effect of the maleic acid derivatives in inhibiting AIMP2-DX2 expression

In order to evaluate the effect of the maleic acid derivatives according to the present invention in inhibiting the expression of AIMP2-DX2, known as an action point of lung cancer, tests were conducted as described below.

### <Example 2-1> Construction of AIMP2-DX2 expression cell line

In order to evaluate the effect of the maleic acid derivatives of the present invention on the inhibition of AIMP2-DX2 expression, the Luciferase Assay System was established. Lung cancer cell line, A549, was purchased from the American Type Culture collection. AIMP2-DX2 gene was inserted into the pGL2 control vector through the HindIII cleavage, such that the luciferase label protein was generated. pGL2-AIMP2-DX2 was transfected into cells using Fugene XD (Roche), and the cells were cultured in RPMI 1640 medium containing 10% FBS and 1% penicillin/streptomycin under conditions of 37□ and 5% CO₂. After culturing for 24 hours, cells were counted, and were dispensed in a 96-well plate at 1 X 10⁴ cells per well.

### <Example 2-2> Inhibition on AIMP2-DX2 expression by tested compounds

The luciferase-AIMP2-DX2-transfected cell line was dispensed in the 96-well plate, cultured for 24 hours, and treated with the maleic acid derivatives (final concentration of 5 µM) in media containing 0.5% serum. After the treatment with the compounds, the cells were cultured for 2 hours, and then treated with lysis reagent (E153A, Promega) diluted with PBS at 100 µL per well, followed by culturing for 15 minutes. The cell lysate was transported on the ELISA plate at 50 µL per well, and analyzed using Luciferase Assay System (Promega). The inhibitory effect on AIMP2-DX2 expression was evaluated by comparing luminescence for each well among the negative control treated with only DMSO and the groups treated with the compounds.

**[Table 2]**

| AIMP2-DX2 inhibitory effect (%, 5 µM) | | | | | |
|---|---|---|---|---|---|
| Example | Inhibitory effect (%) | Example | Inhibitory effect (%) | Example | Inhibitory effect (%) |
| Example 1-1 | 60 | Example 1-2 | 63 | Example 1-3 | 64 |
| Example 1-4 | 62 | Example 1-5 | 41 | Example 1-6 | 51 |
| Example 1-12 | 64 | Example 1-13 | 63 | Example 1-14 | 77 |
| Example 1-15 | 62 | Example 1-16 | 64 | Example 1-17 | 65 |
| Example 1-18 | 63 | Example 1-19 | 60 | Example 1-20 | 89 |
| Example 1-21 | 67 | Example 1-22 | 61 | Example 1-23 | 77 |
| Example 1-25 | 45 | Example 1-26 | 53 | Example 1-29 | 53 |
| Example 1-45 | 29 | Example 1-46 | 42 | Example 1-47 | 41 |
| Example 1-49 | 87 | Example 1-52 | 52 | Example 1-53 | 65 |
| Example 1-54 | 67 | Example 1-55 | 82 | Example 1-56 | 81 |

As shown in Table 2 above, it can be seen that the maleic acid derivatives according to the present invention significantly inhibited AIMP2-DX2 expression at 5 µM. It can be seen that the compounds of Examples 1-1 to 1-4, 1-6, 1-12 to 1-23, 1-26, 1-29, and 1-49 to 1-56 exhibited an inhibitory effect of at least 50%, and especially, the compounds of Examples 1-14, 1-20, 1-23 and 1-49, 1-55, and 1-56 exhibited an excellent inhibitory effect of at least 70%.

Therefore, the maleic acid derivatives of Chemical Formula 1 according to the present invention have an excellent action of inhibiting the expression of AIMP2-DX2, which has been known to be highly expressed in lung cancer patients, and thus can be favorably used in the treatment of lung cancer.

### <Example 3>

### Evaluation on cytotoxicity (MTT) of the maleic acid derivatives on lung cancer cells (H460)

In order to evaluate the cytotoxic effect of the maleic acid derivatives according to the present invention on lung cancer cells, tests were conducted as described below. The maleic acid derivatives according to the present invention were tested by using the compounds of examples 1-14 and 1-20 as representatives thereof.

H460 cells were dispensed in medium containing 0.5% serum at 1 X 10⁴ per well, and were treated with the compounds. MTT solution (5 mg/mL) was diluted 10-fold, and dispensed at 10 µl per well, followed by culturing for 30 minutes. The precipitate was dissolved in 100 µl of DMSO, and the absorbance was measured using a microplate reader (Sunrise, TECAN) at a wavelength of 420 nm.

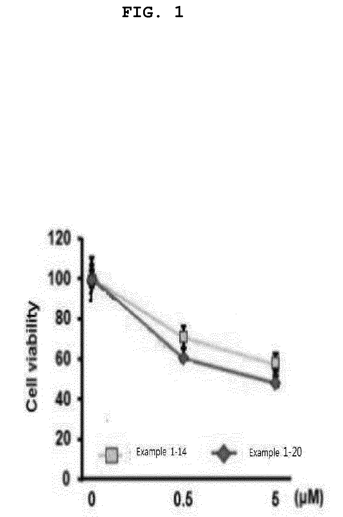

As shown in FIG. 1, the compounds of Examples 1-14 and 1-20 significantly inhibited the proliferation of H460 cells in a dose-dependent manner.

Therefore, the maleic acid derivatives according to the present invention can be used as a therapeutic agent for lung cancer by inhibiting the proliferation of lung cancer cells in a dose-dependent manner.

### <Example 4>

### Evaluation on the effect of the maleic acid derivatives of present invention on AIMP2-DX2 mRNA

In order to evaluate the effect of the maleic acid derivatives according to the present invention on AIMP2-DX2 mRNA, RT-PCR was conducted as follows. The maleic acid derivatives according to the present invention were experimented by using the compounds of Examples 1-14 and 1-20 as representatives thereof.

Total RNA was isolated from cell lines or frozen cancer tissues using Trizol (Invitrogen), and was converted into cDNA using reverse transcriptase of Moloney murine leukemia virus and any hexamer. PCR was conducted using a primer pair of 5'-ATGCCGATGTACCAGGTAAAG-3'(forward) and 5'-CTTAAGGAGCTTGAGGGCCGT-3' (backward) and PCR master mix (Bionia) to measure the full-length AIMP2 transcript (960 bp) and AIMP2-DX2 transcript (753 bp) according to the manufacturer's method. Beta-actin gene was used as a control, and PCR products were separated on 1.5% agarose gel through electrophoresis, and then stained with ethidium bromide.

As shown in FIG. 2, it was suggested that the maleic acid derivatives according to the present invention significantly reduced the expression of AIMP2-DX2 in a dose-dependent manner, and thus the inhibitory effect of AIMP2-DX2 on the proliferation of non-small cellular lung cancer cells is associated with the inhibition of AIMP2-DX2 mRNA.

Therefore, the maleic acid derivatives of Chemical Formula 1 according to the present invention can be effectively used in the treatment of lung cancer by inhibiting AIMP2-DX2 mRNA.

### <Example 5>

### Evaluation on cytotoxicity of the maleic acid derivative according to the present invention on normal cell lines

In order to evaluate the cytotoxicity of the maleic acid derivative (example 1-20 as a representative) according to the present invention on normal cell lines, a cytotoxic test was conducted as follows.

For the normal cell lines, five cell lines, VERO (African green monkey kidney cell line), HFL-1 (human embryonic lung cell line), L929 (NCTC clone 929, mouse fibroblast cell line), NIH 3T3 (mouse embryonic fibroblast cell line), and CHO-K1 (Chinese hamster overy cell line) were used.

For the cytotoxicity, all the cells were seeded at 1 X 10⁴, and for a generalized test for investigating cell viability, the CC₅₀ value, which is the concentration of a compound killing 50% of cells, was measured by cell counting kit-8 assay using tetrazolium salt named WST-8 displaying a pale yellow color.

**[Table 3]**

| Cytotoxicity | | | | | |
|---|---|---|---|---|---|
| Example | CC₅₀ (µM) | | | | |
| | VERO | HFL-1 | L929 | NIH | 3T3 CHO-K1 |
| 1-20 | > 100 | > 100 | > 100 | > 100 | > 100 |

As shown in Table 3, the maleic acid derivative according to the present invention had CC₅₀ values of 100 µM or more on the five kinds of normal cell lines with no observation of cytotoxicity, suggesting its selective cytotoxicity on the lung cancer cell line with anticancer actions.

Therefore, the maleic acid derivative of Chemical Formula 1 according to the present invention acts on only the lung cancer cell line without toxicity on normal cells, and thus can be safely used in the treatment of lung cancer.

### [Industrial Applicability]

As set forth above, the compounds of the present invention possess an excellent action of inhibiting the expression of AIMP2-DX2 and suppressing the proliferation of cancer cells, and thus is effective in cancer prevention or treatment. Further, the compounds of the present invention exhibited no cytotoxicity and was selective only to cancer cells, and thus the safety thereof was verified. Therefore, the compounds of the present invention can be used for the purpose of preventing and treating cancer, leading to their significant industrial applicability.

## Claims

1. A maleic acid derivative represented by Chemical Formula 1 below or a pharmaceutically acceptable salt thereof: 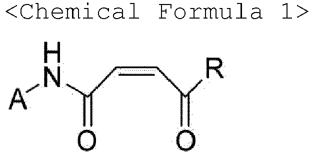 wherein,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group;
when R is OH, the phenyl having a substituent group is excluded from A, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl or phenyl or heteroaryl having a substituent group; and
R is OH, OR¹, NH₂, NHR¹, NR¹R², 5- to 7-membered phenyl having a substituent group, or heteroaryl or heterocycloalkyl containing at least one heteroatom selected from the group consisting of N, O, and S and having a substituent group, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl or phenyl.

2. The maleic acid derivative or the pharmaceutically acceptable salt thereof of claim 1, wherein the maleic acid derivative represented by Chemical Formula 1 is at least one selected from the group consisting of:
(*Z*)-4-oxo-4-(pyridin-2-ylamino)but-2-enoic acid;
(*Z*)-4-[(4-methylpyridin-2-yl)amino]-4-oxobut-2-enoic acid;
(*Z*)-4-oxo-4-(pyridin-3-ylamino)but-2-enoic acid;
(*Z*)-4-[(4,6-dimethylpyridin-2-yl)amino]-4-oxobut-2-enoic acid;
(Z)-4-[(5-chloropyridin-2-yl)amino]-4-oxobut-2-enoic acid;
(*Z*)-4-[(6-bromopyridin-3-yl)amino]-4-oxobut-2-enoic acid;
(*Z*)-4-[(5-methyl-1,3,4-thiadiazol-2-yl)amino]-4-oxobut-2-enoic acid;
(*Z*)-4-oxo-4-[(thiophen-2-ylmethyl)amino]but-2-enoic acid;
(*Z*)-4-[(3-cyanothiophen-2-yl)amino]-4-oxobut-2-enoic acid;
(*Z*)-4-(isoxazol-3-ylamino)-4-oxobut-2-enoic acid;
ethyl (*Z*)-4-[(3,4-dimethylphenyl)amino]-4-oxobut-2-enoate;
(*Z*)-4-oxo-4-(piperidin-1-yl)-N-(*m*-tolyl)but-2-enamide;
(*Z*)-*N*-(3,5-dimethylphenyl)-4-oxo-4-(piperidin-1-yl)but-2-enamide;
(Z)-*N*-(4-methylpyridin-2-yl)-4-oxo-4-(piperidin-1-yl)but-2-enamide;
(*Z*)-4-oxo-4-(piperidin-1-yl)-*N*-(pyridin-2-yl)but-2-enamide;
(*Z*)-4-oxo-4-(piperidin-1-yl)-*N*-(pyridin-3-yl)but-2-enamide;
(*Z*)-N-(4,6-dimethylpyridin-2-yl)-4-oxo-4-(piperidin-1-yl)but-2-enamide;
(Z)-4-morpholino-4-oxo-*N*-(*m*-tolyl)but-2-enamide;
(*Z*)-*N*-(3,5-dimethylphenyl)-4-morpholino-4-oxobut-2-enamide;
(*Z*)-*N*-(4-methylpyridin-2-yl)-4-morpholino-4-oxobut-2-enamide;
(*Z*)-4-oxo-4-(pyrrolidin-1-yl)-*N*-(*m*-tolyl)but-2-enamide;
(*Z*)-*N*-(3,5-dimethylphenyl)-4-oxo-4-(pyrrolidine-1-yl)but-2-enamide;
(*Z*)-*N*-(4-methylpyridin-2-yl)-4-oxo-4-(pyrrolidine-1-yl)but-2-enamide;
(Z)-*N*-(pyridin-2-yl)-4-oxo-4-(pyrrolidine-1-yl)but-2-enamide;
(*Z*)-*N*-(4-bromophenyl)-4-oxo-4-(piperidin-1-yl)but-2-enamide;
(*Z*)-*N*-(4-chlorophenyl)-4-oxo-4-(piperidin-1-yl)but-2-enamide;
(Z)-*N*-(4-bromophenyl)-4-morpholino-4-oxobut-2-enamide;
(Z)-*N*-(5-chloropyridin-2-yl)-4-(piperidin-1-yl)-4-oxobut-2-enamide;
(*Z*)-*N*-(6-bromopyridin-3-yl)-4-(piperidin-1-yl)-4-oxobut-2-enamide;
(*Z*)-*N*-(4-chlorophenyl)-4-oxo-4-(pyrrolidin-1-yl)but-2-enamide;
(*Z*)-*N*-(4-chlorophenyl)-4-morpholino-4-oxobut-2-enamide;
(*Z*)-*N*-(6-bromopyridin-3-yl)-4-morpholino-4-oxobut-2-enamide;
(*Z*)-*N*-(5-chloropyridin-2-yl)-4-morpholino-4-oxobut-2-enamide;
(*Z*)-*N*-(5-chloropyridin-2-yl)-4-oxo-4-(pyrrolidin-1-yl)but-2-enamide;
(*Z*)-*N*-(6-bromopyridin-3-yl)-4-oxo-4-(pyrrolidin-1-yl)but-2-enamide;
(*Z*)-*N*-(5-methyl-1,3,4-thiadiazol-2-yl)-4-oxo-4-(pyrrolidin-1-yl)but-2-enamide;
(*Z*)-4-oxo-4-(pyrrolidin-1-yl)-*N*-(thiophen-2-ylmethyl)but-2-enamide;
(*Z*)-4-oxo-4-(piperidin-1-yl)-*N*-(thiophen-2-ylmethyl)but-2-enamide;
(*Z*)-4-morpholino-4-oxo-*N*-(thiophen-2-ylmethyl)but-2-enamide;
(*Z*)-*N*-(3-cyanothiophen-2-yl)-4-oxo-4-(pyrrolidin-1-yl)but-2-enamide;
(*Z*)-*N*-(3-cyanothiophen-2-yl)-4-morpholino-4-oxobut-2-enamide;
(*Z*)-*N*-(3-cyanothiophen-2-yl)-4-oxo-4-(piperidin-1-yl)but-2-enamide;
(*Z*)-*N*-(3-cyanothiophen-2-yl)-4-oxo-4-(1*H*-pyrazol-1-yl)but-2-enamide;
(*Z*)-*N*-(isoxazol-3-yl)-4-oxo-4-(1*H*-pyrazol-1-yl)but-2-enamide;
(*Z*)-4-[(5-bromopyridin-2-yl)amino]-4-oxobut-2-enoic acid;
(*Z*)-*N*-(5-bromopyridin-2-yl)-4-morpholino-4-oxobut-2-enamide;
(*Z*)-*N*-(5-bromopyridin-2-yl)-4-oxo-4-(pyrrolidin-1-yl)but-2-enamide;
(*Z*)-*N*-(5-bromopyridin-2-yl)-4-oxo-4-(piperidin-1-yl)but-2-enamide;
(*Z*)-4-[(4-chloropyridin-2-yl)amino]-4-oxobut-2-enoic acid;
(*Z*)-*N*-(4-chloropyridin-2-yl)-4-morpholino-4-oxobut-2-enamide;
(*Z*)-*N*-(4-chloropyridin-2-yl)-4-oxo-4-(pyrrolidin-1-yl)but-2-enamide.
(*Z*)-*N*-(4-chloropyridin-2-yl)-4-oxo-4-(piperidin-1-yl)but-2-enamide;
(*Z*)-4-[(4-bromopyridin-2-yl)amino]-4-oxobut-2-enoic acid;
(*Z*)-*N*-(4-bromopyridin-2-yl)-4-morpholino-4-oxobut-2-enamide;
(*Z*)-*N*-(4-bromopyridin-2-yl)-4-oxo-4-(pyrrolidin-1-yl)but-2-enamide;
(*Z*)-4-morpholino-4-oxo-*N*-(4-phenylpyridin-2-yl)-but-2-enamide; and
(*Z*)-4-methyl-2-(4-morpholino-4-oxo-but-2-enamido)pyridine 1-oxide.

3. A method for preparing a maleic acid derivative or the pharmaceutically acceptable salt thereof, by any one selected from the group consisting of Reaction Scheme 1, Reaction Scheme 2, and Reaction Scheme 3 below: 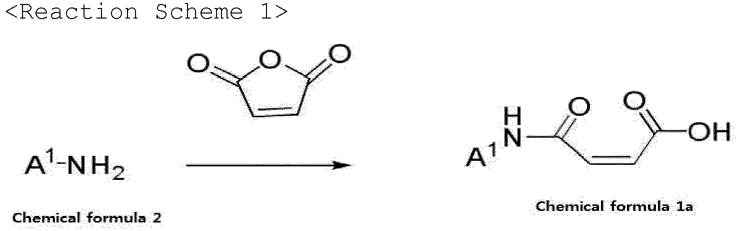 wherein in Chemical Formula 1a and Chemical Formula 2,
A¹ is heteroaryl or heterocycloalkyl containing at least one heteroatom selected from the group consisting N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; 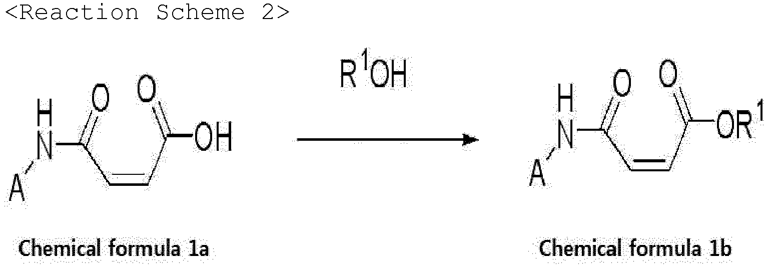 wherein in Chemical Formula 1a and Chemical Formula 1b,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
each R¹ is C₁-C₅ straight- or branched-chain alkyl, or phenyl; and 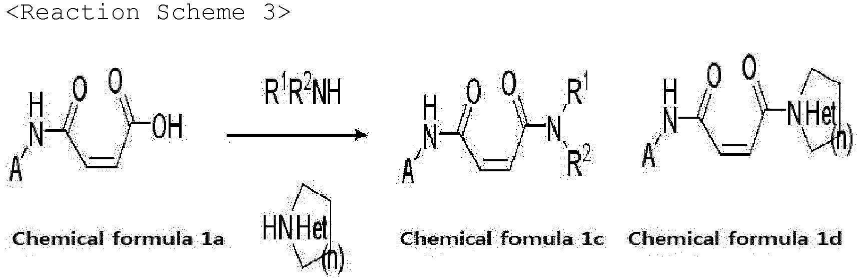 wherein in Chemical Formulas 1a, 1b, and 1c,
A is 5- to 7-membered phenyl having a substituent group, or heteroaryl or heterocycloalkyl containing at least one heteroatom selected from the group consisting of N, O, and S and having a substituent group, or 5- to 7-membered phenyl, heteroaryl, or heterocycloalkyl linked to C₁-C₅ straight- or branched-chain alkyl, wherein the substituent group is unsubstituted, halogen, OH, OR¹, nitro, nitrile, NH₂, NHR¹, NR¹R² COOH, COOR¹, CONH₂, CONHR¹, C₁-C₅ straight- or branched-chain alkyl, phenyl, heteroaryl, or 5- to 7-membered heterocycloalkyl, where R¹ and R² each are C₁-C₅ straight- or branched-chain alkyl, or phenyl or heteroaryl having a substituent group; and
R¹ and R² each are phenyl or heteroaryl having C₁-C₅ straight- or branched-chain alkyl or a substituent group; n is 0 to 2; Het is heteroaryl or heterocycloalkyl having a substituent group containing at least one heteroatom selected from the group consisting of N, O, and S, while including N.

4. A pharmaceutical composition for preventing or treating cancer, the composition containing the maleic acid derivative represented by Chemical Formula 1 of any one of claims 1 and 2 or the pharmaceutically acceptable salt thereof.

5. The pharmaceutical composition of claim 4, wherein the cancer is at least one disease selected from the group consisting of breast cancer, large intestine cancer, lung cancer, small cell lung cancer, gastric cancer, liver cancer, blood cancer, bone cancer, pancreatic cancer, skin cancer, head or neck cancer, cutaneous or intraocular melanoma, uterine cancer, ovarian cancer, rectal cancer, anal cancer, colon cancer, breast cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid carcinoma, adrenal cancer, soft tissue sarcoma, urethral cancer, penile cancer, prostate cancer, chronic or acute leukemia, lymphocyte lymphoma, bladder cancer, kidney cancer, ureter cancer, renal cell carcinoma, renal pelvic carcinoma, CNS tumor, primary CNS lymphoma, spinal cord tumor, brain stem glioma, and pituitary adenoma.

6. The pharmaceutical composition of claim 4, wherein the cancer is lung cancer.

7. A method for preventing or treating cancer, the method comprising administering the maleic acid derivative of claim 1 or the pharmaceutically acceptable salt thereof to a subject in need thereof.

8. Use of the maleic acid derivative or the pharmaceutically acceptable salt thereof of claim 1 for the preparation of an agent for preventing or treating cancer.
